# EUROPEAN PATENT SPECIFICATION

(11) **EP 1 481 007 B1**
(45) Date of publication and mention of the grant of the patent: **24.01.2007**
(21) Application number: 03706165.2
(22) Date of filing: 04.03.2003
(51) Int. Cl.: C07K 14/47, G01N 33/68, C12Q 1/68, A01K 67/027, C12N 5/10, C12N 15/12, A61P 25/28

(54) **SPHERON COMPONENTS USEFUL IN DETERMINING COMPOUNDS CAPABLE OF TREATING SYMPTOMS OF ALZHEIMER'S DISEASE, TREATMENTS AND ANIMAL MODELS PRODUCED THEREFROM**
SPHERON-KOMPONENTEN FÜR DIE BESTIMMUNG VON VERBINDUNGEN, DIE SYMPTOME DER ALZHEIMER KRANKHEIT BEHANDELN KÖNNEN, IHRE BEHANDLUNGEN UND TIERMODELLE
COMPOSANTS DE SPHERON DES PLUS UTILES S'AGISSANT D'IDENTIFIER DES COMPOSES CAPABLES DE TRAITER LES SYMPTOMES DE LA MALADIE D'ALZHEIMER, TRAITEMENTS ET MODELES ANIMAUX PRODUITS A L'AIDE DE CES COMPOSANTS

(30) Priority: 04.03.2002 US 361302 P
(43) Date of publication of application: 01.12.2004
(73) Proprietor: NYMOX CORPORATION, St. Laurent Québec H4M 3V2 (CA)
(72) Inventor: AVERBACK, Paul, Beaconsfield, Québec H9W 4J1 (CA)
(74) Representative: Plougmann & Vingtoft A/S
(86) International application number: PCT/CA2003/000295
(87) International publication number: WO 2003/074558

(56) References cited:
- WO-A-98/34643
- WO-A-98/52898
- WO-A-99/55839
- GB-A- 2 352 631
- BASAK A ET AL.: "Inhibitory Specificty and Potency of proSAAS-derived Peptides toward Proprotein Convertase 1" THE JOURNAL OF BIOLOGICAL CHEMISTRY, vol. 276, no. 35, 31 August 2001 (2001-08-31), pages 32720-32728, XP002251990
- CAMERON A ET AL.: "Polyarginines Are Potent Furin Inhibitors" THE JOURNAL OF BIOLOGICAL CHEMISTRY, vol. 275, no. 47, 24 November 2000 (2000-11-24), pages 36741-36749, XP002251991
- FRICKER LD ET AL.: "Identification and Characterization of proSAAS, a Granin-Like Neuroendocrine Peptide Precursor that Inhibits Prohormone Processing" THE JOURNAL OF NEUROSCIENCE, vol. 20, no. 2, 15 January 2000 (2000-01-15), pages 639-648, XP002251992
- QIAN Y ET AL.: "The C-terminal Region of proSAAS Is a Potent Inhibitor of Prohormone Convertase 1" THE JOURNAL OF BIOLOGICAL CHEMISTRY, vol. 275, no. 31, 4 August 2000 (2000-08-04), pages 23596-23601, XP002251993

## Description

### BACKGROUND OF THE INVENTION

### 1. Field of the Invention

The present invention relates to purified protein components of spherons that are useful for screening compounds capable of preventing and/ or ameliorating symptoms associated with cerebral amyloidosis, dementia, or Alzheimer's Disease. The invention also relates to methods of making an Alzheimer's Disease or dementia non-human animal model, the non-human animal model produced therefrom, and to a method of using the animal model to screen for effective Alzheimer's Disease therapies. The invention further relates to methods and compositions that prevent the release of, or reduce the concentration of pro-SAAS protein or fragments thereof, or block, compete with, or attenuate the effects of increased amounts of proSAAS protein or fragments thereof, in the brain.

### 2. Description of Related Art

Classified under the rubric "amyloidosis" are a number of pathological conditions characterized by the deposition of abnormal fibrils ("amyloid fibrils") in extracellular spaces. The amyloid fibril, in turn, represents a final common pathway for a diverse array of proteins. Regardless of their biochemical composition, however, all types of amyloid fibrils share (a) a -pleated sheet structure, (b) green birefringence under polarized light after staining with Congo Red dye, and (c) a fibrillar morphology that has a typical electron-microscopic appearance.

The deposition of amyloid fibrils can affect several organs in the systemic forms of the disorder, exemplified by familial Mediterranean fever, familial amyloid polyneuropathy and systemic amyloidosis, or it can be restricted to one organ in localized forms. Among the latter are conditions classified under the rubric "cerebral amyloidosis," which covers the Alzheimer group of diseases, namely, Alzheimer's disease (pre-senile dementia, senile dementia); Alzheimer's disease associated with Down' syndrome; Alzheimer's disease associated with other central-nervous-system diseases, such as Parkinson's disorder; and congophilic angiopathy (associated or not associated with Alzheimer's disease). Alzheimer's disease in general is an incurable brain disease affecting middle aged and elderly people throughout the world. According to most recent estimates, it is the fourth or fifth leading cause of death among North Americans, and is responsible for inestimable personal and social tragedy, loss of productivity, and custodial burden to society. There is presently no widely-accepted effective treatment for Alzheimer's disease.

The principle symptom (manifestation) of Alzheimer's disease is the loss of higher mental faculties, typified by the loss of memory and behavior referred to as "dementia." Dementia is a symptom or syndrome that can be seen in many brain diseases other than Alzheimer's disease, such as stroke, encephalitis and metabolic diseases. Since memory loss and dementia are relatively nonspecific symptoms, a certain and specific definition of Alzheimer's disease is based on the characteristic microscopic state of the brain, described initially by Marinesco, Alzheimer and others. See Alzheimer, A., *Allgemeine Zeitschrift für Psychiatrie* **64:146-148** (1907); Marinesco, G., *Comptes Rendus des Seances de la Societe de Biologie et ses Filiales* **70:606-608** (1911).

The particular microscopic features that are accepted indicators of Alzheimer's disease, and that separate Alzheimer's disease from other causes of dementia, are the accumulation of large numbers of brain lesions referred to as senile or amyloid plaques and neurofibrillary tangles. Senile or amyloid plaques are spherical, ranging from 10 to 200 micrometres in diameter, and while found only occasionally in aged adult cerebral cortex, are found in large numbers in Alzheimer-affected cortex. These lesions, when found in suitable quantity in a brain sample, are the definitive criteria for the diagnosis of Alzheimer's disease.

Amyloid plaques in large quantities are essentially found only in the Alzheimer group of diseases, whereas neurofibrillary tangles are nonspecific and are found in at least ten other neurological diseases. *See* Corsellis, J.A.N., GREENFIELD'S NEUROPATHOLOGY 951-1025 (4th ed. 1984) (Edward Arnold, London). Individual amyloid plaques have roughly 1000 x the volume of individual neurofibrillary tangles. True measurements of total brain amyloid plaque and neurofibrillary content are not available, but on the above basis it is likely that the volume of abnormal brain tissue occupied by amyloid plaques is many hundreds of times that of neurofibrillary tangles. The essential feature of the amyloid plaque is the presence of amyloid fibrils, which are a congophilic red-green birefringent microfibrillar material. Corsellis, *loc. cit.*

A microscopic structure referred to as the dense microsphere (DMS) is known to exist in normal brain and in brain affected by Alzheimer's disease. *See* Averback, *Acta Neuropathol.* **61:148-52** (1983); results confirmed by Hara, *J*. *Neuropath. Exp. Neurol.* **45(2):169-178** (1986). The DMS more recently have been called "spherons." "Spherons" in the context of the present invention therefore will denote the same thing as DMS, and refer to the spherical microscopic structure that exists in normal brain.

Some specialists believe that spherons are linked to cerebral amyloid plaques as the source of, or as a precursor to, the cerebral amyloid characteristic of Alzheimer's disease and related conditions. Evidence for the existence of spherons comes from microscopic histological section studies of fixed whole brain tissue. The spherons are observed as randomly dispersed, highly infrequent structures numbering ~10²/mm³.

Various components of spherons (e.g., "DMS") have previously been identified. It is known that the disintegration of spherons in the brain parenchyma is associated with the onset and progression of cerebral amyloid plaque formation that is characteristic of Alzheimer's disease and related conditions. More specifically, disintegration of spherons releases protein and non-protein components, or mixtures of protein and non-protein components. A portion of the spheron components form cerebral amyloid plaques, while other portions either remain in the brain, or are removed from the brain parenchyma via circulating bodily fluids, including cerebrospinal fluid, serum and the like.

It also is believed that spherons undergo a growth and bursting cycle bringing about a cataclysmic cascade of spheron bursts, and subsequent brain injury. As disclosed in U.S. Patent No. 6,130,221, an important mechanism of initiation and promotion of spheron disruption has been discovered that involves a distinctive autocatalytic phenomenon, whereby the disruption, degeneration, and evolution of an individual spheron into an individual cerebral amyloid plaque provides the stimulus for a group or field of other spherons to disrupt, degenerate and evolve in a recurring set of waves. This unchecked, autocatalytic phenomenon causes an exponential growth pattern: small, perhaps statistically insignificant differences (between individual brains) in starting numbers of disrupted spherons *in situ* evolve into statistically significant differences after generations of the cycle. For example, if all other factors were equal, a subject having an initial group of 100 spheron bursts would not be statistically or symptomatically different from a second subject having an initial group of 98 spheron bursts. However, if over time each of the initial spheron initiated 10 subsequent spherons to disrupt, each of which in turn initiated 10 subsequent spheron disruptions, then group 1 after 20 generations would have 2 x 10²⁰ more disrupted spherons than group 2, which is significant.

### SUMMARY OF THE INVENTION

There remains a need in the art for new treatments for treating symptoms of AD not directly attributable to neurotransmitter deficits or to amyloid plaque formation. There also exists a need to develop methods for identifying therapies useful in treating such symptoms, as well as non-human animal models useful in screening for therapies useful in treating these symptoms. The present invention satisfies these needs.

The present invention is premised in part on the surprising discovery that peptides containing amino acid sequences corresponding to components of spherons that are not directly attributable to amyloid plaque formation are toxic to neuronal cells, and are believed to cause other deleterious effects, including neuronal dysfunction. The invention also is premised in part on the discovery that these particular components, when released from the spherons, damage the brain in a manner that is distinguishable from the amyloid-producing effects.

The invention also is premised on the discovery that these particular components in the concentrations present when released from the spherons, provide greatly increased activity on proprotein convertase 1 (PC1) inhibition, (as well as increased activity on proprotein convertases PC2-PC8), and furin, leading to decreased adrenocorticotrophic hormone (ACTH), insulin, _MSH, _endorphin, and enkephalin production, as well as decreased or otherwise altered thyrotropin-releasing hormone (TRF), dynorphin, pro-insulin, pro-glucagon, pro glucagon-like peptide (GLP), pro-somatostatin, pro-pancreatic peptide, pro-GHRH, neuropeptide melamin-concentrating hormone, NEI, neurotensin, opioid peptides, and other peptides and neuronal dysfunction. These components also provide increased neuropeptides from proSAAS domains. All of the above are believed to lead to a cascade of effects including but not limited to derangements of glucose production (insulin and ACTH), derangements of corticosteroid pathways (ACTH), derangements of thyroid pathways (TRH), derangements of enkephalins, derangements of other significant bioactive cerebral molecules and molecular pathways, as well as derangements of glucagon, GLP, somatostatin, pancreatic peptide, GHRH, neuropeptide melanin-concentrating hormone, NEI, neurotensin, and opioid peptides. In addition, greatly increased activity on proprotein convertase inhibition will affect the post-translational processing of proenzymes such as proBACE (beta-site APP cleaving enzyme, also known as beta-secretase) involved in the post translational processing, modification and cleavage of amyloid precursor protein (APP). These above diverse biochemical pathway imbalances are well known to be disease producing and symptom producing. These imbalances are well known to produce behavioral, mental, and cognitive symptoms, the latter being similar to those found in neurodegenerative disorders, such as dementia and dementia associated with various types and Alzheimer's disease.

In accordance with a feature of an embodiment of the present invention, there is provided a method of identifying compounds useful in treating or ameliorating a neurological condition comprising:
contacting a test compound with a spheron component peptide other than a spheron component responsible for forming amyloid plaque, the spheron component peptide being selected from the group consisting of:
   GEAAGAVQELAR (SEQ ID NO. 1);
   GLSAASPPLAETGAPR (SEQ ID NO. 2);
   ARAEAQEAEDQQAR (SEQ ID NO. 3);
   ALAHLLEAERQER (SEQ ID NO. 5);
   AADHDVGSELPPEGVLGALLR (SEQ ID NO. 6);
   LETPAPQVPAR (SEQ ID NO. 7);
   ILAGSADSEGVAAPR (SEQ ID NO. 8);
   ARPVKEPRGLSAASPPLAETGAPRRF (SEQ ID NO. 9);
   ARPVKEP (SEQ ID NO. 10);
   GLSAASPPLAETGAPRRF (SEQ ID NO. 11);
   AADHDVGSELPPEGVLGALLRVKRLETPAPQVPA (SEQ ID NO. 12);
   AADHDVGSELPPEGVLGALLRV (SEQ ID NO. 13);
   LETPAPQVPA (SEQ ID NO. 14);
   RRSVPRGEAAG (SEQ ID NO. 15);
   VLAQLLRVWGAPRNSD (SEQ ID NO. 16);
   PALGLDDDPDAPAAQLAR (SEQ ID NO. 17);
   LARALLRARLDPAALAA (SEQ ID NO. 18);
   QLVPAPVPAAALRPRPPVYDD (SEQ ID NO. 19);
   GPAGPDAEEAGDE (SEQ ID NO. 20);
   TPDVDPELLRYLLGR (SEQ ID NO. 21); and
   LLRVKR (SEQ ID NO. 22);
and determining whether the test compound binds to, antagonizes, or competes with the spheron component peptide,
with the proviso that if said test compound is administered to an animal that has been transfected with a transformed, transfected or infected cell comprising the spheron component peptide, said animal is a non-human animal.

In accordance with another feature of an embodiment of the present invention, there is provided a composition as defined in claim 16

In accordance with another feature of an embodiment of the invention, there is provided an Alzheimer's Disease symptom non-human animal model, or non-human test animal, a method of making the non-human animal, model or non-human test animal, and to a method of using the non-human animal model or non-human test animal to screen for effective therapies for treating or ameliorating brain damage caused by spheron components other than a spheron component directly responsible for forming amyloid plaque. The non-human animal model comprises an animal having a gene inserted into the brain thereof, whereby the gene over-expresses one or more spheron component peptides selected from the group defined above. The non-human test animal comprises a non-human animal having one or more of the spheron component peptides selected from the group defined above administered thereto, whereby administration may be by intrathecal, intravenous, intra cerebral ventricular, or other administration routes. The method of making the non-human animal model therefore includes preparing a gene that expresses one or more of spheron component peptides selected from the group defined above, incorporating the gene into the brain of a non-human animal (*e*.*g*. by a transgenic mechanism, according to methods well known in the art), and inducing the gene to ver-express the spheron component peptide. The method of making the non-human test animal includes administering to the animal one or more of the spheron component peptides selected from the group defined above.

The method of using the non-human animal model or non-human test animal includes preparing a group of non-human test animals, or preparing a group of non-human animals having a gene in the brain thereof that over-expresses one or more of spheron component peptides selected from the group defined above, and then inducing the gene to over-express the spheron component peptide. The method includes administering to a select group of non-human animals a test compound, sacrificing the non-human animals, and then measuring the amount of isolated spheron component peptides present in the sacrificed non-human animal's brain, and/ or measuring the percentage of viable cells at or around the locus of the gene and/ or measuring the injury of the brain in relation to the gene over-expression, and/or measuring any altered brain function, behavior, etc. in the animal. The method concludes by selecting those test compounds that reduce the amount of isolated spheron component peptides, when compared to controls, and/ or by selecting those test compounds that yield a higher percentage of viable cells at or around the locus of the gene, when compared to controls having no test compound administered thereto, and/ or by selecting those test compounds that yield less brain injury in relation to the gene over-expression compared to controls, and/or by selecting those test compounds that result in improved brain function, behavior, etc. compared to controls.

These and other features of various embodiments of the present invention will be readily apparent to one of ordinary skill in the art upon a review of the detailed description of the invention, including the examples.

### DETAILED DESCRIPTION OF THE PREFERRED EMBODIMENTS

Terms and phrases used herein are defined as set forth below unless otherwise specified. Unless defined otherwise, all technical and scientific terms used herein have the same meanings as commonly understood by

Throughout this description, the phrase "Alzheimer's disease and related conditions" denotes conditions classified under the rubric "cerebral amyloidosis." Such conditions include, but are not limited to Alzheimer's disease [pre-senile dementia, senile dementia]; Alzheimer's disease associated with Down's syndrome; Alzheimer's disease associated with other central-nervous-system diseases, such as Parkinson's disorder; congophilic angiopathy [associated or not associated with Alzheimer's disease], and other dimentia such as Frontotemporal Dimentia (FTD), mild cognitive impairment, and the like. Throughout this description, the phrase "DMS components" or "spheron components" denotes any component of DMS, protein, non-protein, or mixtures of protein and non-protein, originating from the internal or central region or from the outer membrane portion of DMS. Throughout this description, the phrase "disrupting" or "digesting a suspension comprising DMS" denotes any process whereby DMS are broken down into DMS components.

"Spheron components" are referred to herein as components of spherons, (formerly DMS), and include those specifically described herein, as well as any and all components described in U.S. Patent Nos. 6,309,892, and

As used herein and in the appended claims, the singular forms "*a*," "*an*," and "the" include plural reference unless the context clearly dictates otherwise. Thus, for example, a reference to "a host cell" includes a plurality of such host cells, and a reference to "an antibody" is a reference to one or more antibodies and equivalents thereof known to those skilled in the art, and so forth.

Amino acids and amino acid residues described herein may be referred to according to the accepted one or three-letter code provided in the table below. Unless otherwise specified, these amino acids or residues are of the naturally occurring L stereoisomer form.

| Amino Acid | One-Letter Symbol | Three-Letter Symbol |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

A "composition comprising a given molecule" (e.g., antibody, bispecific antibody, or diabody) or a "composition comprising a given amino acid sequence," as these terms are used herein, refer broadly to any composition containing the given molecule, polynucleotide or amino acid sequence. The composition may comprise a dry formulation, an aqueous solution, a solution in a non-aqueous solvent, or a sterile composition. The compositions may be stored in freeze-dried form and may be associated with a stabilizing agent such as a carbohydrate. In hybridizations and other applications, the compositions may be deployed in an aqueous solution containing salts, e.g., NaCl, detergents, e.g.,sodium dodecyl sulfate (SDS), and other components, e.g., Denhardt's solution, dry milk, salmon sperm DNA, etc.

Throughout this description and in the appended claims, reference to a "peptide," a "spheron component peptide," and specific amino acid sequences include those sequences specifically identified.

Throughout this description, the expressions "specific binding" or "specifically binding," "binding," "binds," and/or "recognizes" refer to the interaction between a molecule, protein or peptide and an agonist, an antibody, or an antagonist. The interaction is dependent upon the presence of a particular structure of the protein, e.g., the antigenic determinant or epitope, recognized by the binding molecule. For example, if an antibody is specific for epitope "A," the presence of a polypeptide containing the epitope A, or the presence of free unlabeled A, in a reaction containing free labeled A and the antibody will reduce the amount of labeled A that binds to the antibody.

The present inventor has found that certain spheron component peptides that are not the spheron component peptides directly attributable to amyloid plaque formation, are, *inter alia,* cytotoxic. The inventor has found that these particular spheron component peptides reduce the viability of brain cells. The cytotoxic effects of these spheron component peptides, when released in the brain of an non-human animal, are believed to cause brain damage, neuronal dysfunction, and Alzheimer's Disease symptoms that are distinguishable from amyloid-producing effects. Based on this discovery, and not intending on being bound by any theory of production or operation, the present inventor believes that reducing and/ or preventing the cytotoxicity and/ or neuronal dysfunction of the spheron component peptides will prevent and/or ameliorate at least one or more symptoms of Alzheimer's Disease, or dementia associated with cerebral amyloidosis.

The present inventor also believes that the presence of these particular spheron components in the concentrations in which they are released from the spherons may be the cause of many other adverse affects. The spheron component peptides described herein are similar to, and consistent with portions of a protein referred to as proSAAS, which is known as a granin-like neuroendocrine peptide precursor. Pro SAAS is active at a tissue concentration of 1nM. ProSAAS derived from spherons is present in the brain in spherons at a brain concentration much higher than 1nM. At these selected concentrations, proSAAS and the spheron component peptides described herein, provide greatly increased activity on proprotein convertase 1 (PC1) inhibition, (as well as increased activity on proprotein convertases PC2-PC8), and furin, leading to decreased adrenocorticotrophic hormone (ACTH), insulin, _MSH, _endorphin, and enkephalin production, as well as decreased or otherwise altered thyrotropin-releasing hormone (TRF), dynorphin, pro-insulin, pro-glucagon, pro-glucagon-like peptide (GLP) pro-somatostatin, pro-pancreatic peptide, pro-GHRH, neuropeptide melamin-concentrating hormone, NEI, neurotensin, opioid peptides, and other peptides, and neuronal dysfunction. In addition, greatly increased activity on proprotein convertase inhibition will affect the post-translational processing of proenzymes such as proBACE (beta-site APP cleaving enzyme, also known as beta-secretase) involved in the post-translational processing, modification and cleavage of amyloid precursor protein (APP). These components also provide increased neuropeptides from proSAAS domains. All of the above are believed to lead to a cascade of effects including but not limited to derangements of glucose production (insulin and ACTH), derangements of corticosteroid pathways (ACTH), derangements of thyroid pathways (TRH), derangements of enkephalins, derangements of other significant bioactive cerebral molecules and molecular pathways, as well as derangements of glucagon, somatostatin, pancreatic peptide, GHRH, neuropeptide melanin-concentrating hormone, NEI, neurotensin, and opioid peptides. These above diverse biochemical pathway imbalances are well known to be disease producing and symptom producing. These imbalances are well known to produce behavioral and mental and cognitive symptoms, the latter being similar to those found in neurodegenerative disorders, such as dementia of various types and Alzheimer's disease.

The present inventor therefore believes that abnormally elevated proSAAS concentrations derived from spherons will be pharmacologically blocked by compounds that will be effective in ameliorating or attenuating the harmful effects of the abnormally elevated quantities of proSAAS in the brain. It is further envisaged that abnormally elevated proSAAS concentrations derived from spherons will be pharmacologically blocked by compounds that will be effective in ameliorating or attenuating the harmful effects of the decreased breakdown of pro-opiomelanocortin (POMC) brought about by the abnormally elevated quantities of proSAAS in the brain in relation to the spherons. It is further envisaged that abnormally elevated proSAAS concentrations derived from spherons will be pharmacologically blocked by compounds that will be effective in ameliorating or attenuating the harmful effects of the elevated levels of molecules and pathways related to ACTH, insulin, and enkephalins, brought about by the abnormally elevated quantities of proSAAS in the brain.

The present inventor also believes that the presence of spheron component peptides that are similar to and consistent with portions of proSAAS in live tissue not only is an indication of the disruption of spherons, and hence the onset of cerebral amyloidosis, as previously reported, but it also is toxic insofar as it causes other live tissue cell death. The presence of these spheron component peptides also will have greatly increased activity on PC1 and PC2 inhibition, leading to decreased ACTH, insulin, and enkephalin production; as well as decreased TRF, dynorphin, and other peptides. The presence of proSAAS or fragments thereof at these concentrations also will lead to increased neuropeptides from proSAAS domains.

The present inventor believes that spheron component peptides present in live mammalian brain tissue is a marker for cerebral amyloidosis, and that it exacerbates cerebral amyloidosis by causing cell necrosis in the live tissue in which it exists. Accordingly, it is believed that, as a mammal becomes inflicted with cerebral amyloidosis, spherons begin to disrupt, burst, and/ or otherwise disintegrate thereby producing spheron component peptides. The present inventor believes that some of the spheron component peptides so produced are believed to be precursors to harmful amyloid protein, while other spheron component peptides begin destroying other live tissue without forming amyloid (e.g., other nerve cells and brain tissue) in the vicinity of the spheron component, thereby exacerbating the progression of the disease. These conditions are in addition to the other conditions described above that are caused by the increased abnormally high concentrations of proSAAS, or fragments thereof, that are believed to be released from the spherons.

The invention therefore relates to a method of treating or ameliorating brain damage and other disorders that comprises administering to an animal in need thereof, a compound that prevents the cytotoxic effects, (and other deleterious effects described herein or later discovered), of a spheron component peptide other than a spheron component directly responsible for forming amyloid plaque, the spheron component peptide being one or more component selected from,
GEAAGAVQELAR (SEQ ID NO.1);
GLSAASPPLAETGAPR (SEQ ID NO. 2);
ARAEAQEAEDQQAR (SEQ ID NO. 3);
ALAHLLEAERQER (SEQ ID NO. 5);
AADHDVGSELPPEGVLGALLR (SEQ ID NO. 6);
LETPAPQVPAR (SEQ ID NO. 7);
ILAGSADSEGVAAPR (SEQ ID NO. 8);
ARPVKEPRGLSAASPPLAETGAPRRF (SEQ ID NO. 9);
ARPVKEP (SEQ ID NO. 10);
GLSAASPPLAETGAPRRF (SEQ ID NO. 11);
AADHDVGSELPPEGVLGALLRVKRLETPAPQVPA (SEQ ID NO. 12);
AADHDVGSELPPEGVLGALLRV (SEQ ID NO. 13);
LETPAPQVPA (SEQ ID NO. 14);
RRSVPRGEAAG (SEQ ID NO. 15);
VLAQLLRVWGAPRNSD (SEQ ID NO. 16);
PALGLDDDPDAPAAQLAR (SEQ ID NO. 17);
LARALLRARLDPAALAA (SEQ ID NO. 18);
QLVPAPVPAAALRPRPPVYDD (SEQ ID NO. 19);
GPAGPDAEEAGDE (SEQ ID NO. 20);
TPDVDPELLRYLLGR (SEQ ID NO. 21); and
LLRVKR (SEQ ID NO. 22).

The methods and compositions of the invention are effective in treating disorders, including Alzheimer's disease, vascular dementia or multi-infarct dementia, dementia with Lewy bodies, Parkinson's disease, Pick's disease, Frontotemporal Dementia (FTD), and mild cognitive impairment. The inventive composition therefore includes a pharmaceutically effective amount of a compound that counteracts the effects of the release of proSAAS or peptide fragments thereof by spherons in the brain. Counteracting the effects of such spheron components can be accomplished in many ways, but the end result is to render such spheron components inactive (or by off-setting their deleterious effects) in one form or another. The composition preferably counteracts the effects of: (i) abnormally inhibited activity of proprotein convertases 1-8 (PC1-PC8) and furin in the brain or in other neuroendocrine tissues caused by the release of proSAAS or peptide fragments thereof from spherons in the brain; (ii) abnormally decreased adrenocorticotrophic hormone (ACTH) caused by the release of proSAAS or peptide fragments thereof from spherons in the brain; (iii) abnormally decreased insulin caused by the release of proSAAS or peptide fragments thereof from spherons in the brain; (iv) abnormally decreased enkephalins caused by the release of proSAAS or peptide fragments thereof from spherons in the brain; (v) abnormally decreased thyrotropin-releasing hormone (TRH) caused by the release of proSAAS or peptide fragments thereof from spherons in the brain; (vi) abnormally decreased dynorphin caused by the release of proSAAS or peptide fragments thereof from spherons in the brain; and (vii) abnormally increased effects such as neurotensin effects and cell signaling effects caused by abnormally increased other proSAAS fragments described herein.

The compositions, methods, and compounds of the invention also are effective in reducing or counteracting the inhibition of PC1 or other convertases (e.g., PC2-PC8, SPC3) and furin, caused by the release of proSAAS or peptide fragments thereof from spherons in the brain. It is preferred that the compounds, compositions, and methods of the invention counteract the effects of the release of proSAAS or peptide fragments from spherons in the brain by increasing or otherwise altering: (i) cerebral ACTH; (ii) cerebral insulin; (iii) cerebral enkephalins; (iv) cerebral TRH; (v) cerebral dynorphin; (vi) cerebral_MSH and _endorphin; (vii) cerebral pro-insulin; (viii) cerebral pro-glucagon; (ix) cerebral pro-somatostatin; (x) cerebral pro-pancreatic peptide; (xi) cerebral pro-GHRH; (xii) cerebral neuropeptide melamin-concentrating hormone; (xiii) cerebral NEI; (xiv) cerebral neurotensin; and (xv) cerebral opioid peptides, and the like.

The above-mentioned levels can be increased by adding the specific component listed above, and consequently, the compositions and methods of the invention include one or more of the above-listed components, such as a proprotein convertase, fragment, domain or subunit thereof, whereby the proprotein convertase has an affinity for the peptide fragments released from spherons in the brain SEQ ID NOs. 1-3, 5-22 The compositions also may include an antibody, antibody fragment or short chain antibody that has an affinity for the peptide fragments released from spherons in the brain SEQ ID NOs. 1-3, 5-22

As mentioned previously, the spheron component peptides listed above are similar to, and consistent with all or portions of a protein referred to as pro-SAAS, which is known as a granin-like neuroendocrine peptide precursor. The identification and characterization of pro-SAAS is described in Fricker, L.D., *et al.,* "Identification and Characterization of pro-SAAS, a Granin-Like Neuroendocrine Peptide Precursor that Inhibits Prohormone Processing," J. *Neurosci,* 20(2):639-648 (2000). It is believed that pro-SAAS is linked to obesity in animals. It was not heretofore known that proSAAS or any of the possible peptides that form a portion of pro-SAAS had any relation to spherons, or any cytotoxic affect on brain cells or any relation to Alzheimer's disease, dementia, or cerebral amyloidosis. It also was not heretofore known that any of the possible peptides that form all or a portion of pro-SAAS were released by, or otherwise derived from spherons in concentrations high enough to cause any of a number deleterious effects.

In accordance with another feature of an embodiment of the present invention, there is provided a composition comprising a compound that does one or more of: (i) impedes or prevents the release of a peptide fragment of the present invention; (ii) binds to, inhibits, antagonizes or competes with a peptide fragment of the present invention; or (iii) reduces or prevents the cytotoxic or other deleterious effects of a peptide fragment of the present invention where by the peptide fragment thereof is a spheron component peptide other than a spheron component responsible for forming amyloid plaque, the spheron component peptide being one or more of the components selected from the above group.

The spheron component peptides described above can be isolated and purified using techniques known and described in the aforementioned articles, as well as those described in U.S. Patent Nos. 5,525,339, and 6,309,892. Spherons may be derived form mammalian brain tissue and characterized, in essentially homogeneous form, by a range of diameters from about 0.1_m to about 15 _m, and by certain stainability properties. In this regard, "homogeneous" means that the spherons represent the only structure discernible in the subject composition at the light-microscopic level. For example, the microspheric bodies of the present invention are homogeneously electron-dense when stained with osmium and lead, and can be visualized by thin-section electron microscopy; under optical microscopic examination, they appear eosinophilic and phloxinophilic, and are nonbirefringent when stained with Congo Red. When the microspheric bodies of the present invention are disrupted or disintegrated or digested, a material is produced that displays congophilic birefringence; that is, when stained with Congo Red the material becomes optically anisotropic to the extent of splitting an incident light wave into two waves with mutually perpendicular vibrational planes. Amyloid protein can also be detected by immunological labeling methods in spherons.

The spherical, intracellular spherons typically are found in human and other mammalian brains in gray-matter neuropil, where the spherical structures are enclosed in tiny, neuronal cellular processes. Spherons usually are solitary, non-perikayal and non-confluent, and are not easily found in cerebellum or in white matter. With regard to inter-spheron distances, the spatial distribution of spherons in gray matter regions is random. Compositions of spherons in homogeneous form can be produced by extraction to produce homogeneous samples of globular bodies according to procedures described in U.S. Pat. Nos. 4,816,416 and 5,231,170, Spherons contain anyloid.

The homogeneous composition of spherons prepared according to the above-described procedure can be disrupted by procedures described in the aforementioned U.S. Pat. Nos. 4,816,416 and 5,231,170, and then subjected to differential gradient centrifugation. Materials isolated in distinct sedimentation layers are stained with Congo Red. The present inventor believes that the spheron components are primarily responsible for the formation of cerebral amyloid plaques. The protein components of spheron membranes can be isolated by conventional extraction methods. Further analysis of spheron components can be accomplished by extraction of such components and by conventional methods such as chromatography that are well-known to those of ordinary skill in the art.

Spherons can be treated by a variety of methods to yield spheron components suitable for use according to the present invention. Exemplary of these methods are: (a) PAGE buffer solutions including TRIS, glycerol, .beta.-mercaptoethanol, bromophenol blue and sodium dodecyl sulfate (SDS), (b) ultrasonication and (c) other proteolytic treatments such as treating with various combinations of 0.25M acetic acid, 6M guanidine HCl, formic acid, 6M urea, pepsin and cyanogen bromide. The resulting homogeneous composition of spheron components can be further refined by isolating the components according to their molecular weight by polyacrylamide gel electrophoresis (PAGE) or according to the degree of their hydrophobicity by reverse phase high performance liquid chromatography (rpHPLC). Spheron components isolated by PAGE can be further characterized as either discrete migrating or non-migrating components. Spheron components also can be extracted from cerebrospinal fluid and other bodily fluids using the extraction procedures described above.

Among the numerous spheron components extracted in accordance with the procedures outlined herein, the particular spheron component peptides that are cytotoxic in tissue culture, and are believed to be cytotoxic in *vivo* to neuronal cells are listed below:

Spheron Component peptide #1 [SEQ ID NO 1]
GEAAGAVQELAR
Gly-Glu-Ala-Ala-Gly-Ala-Val-Gln-Glu-Leu-Ala-Arg

Spheron Component peptide #2 [SEQ ID NO 2]
GLSAASPPLAETGAPR
Gly-Leu-Ser-Ala-Ala-Ser-Pro-Pro-Leu-Ala-Glu-Thr-Gly-Ala-Pro-Arg

Spheron Component peptide #3 [SEQ ID NO 3]
ARAEAQEAEDQQAR
Ala-Arg-Ala-Glu-Ala-Gln-Glu-Ala-Glu-Asp-Gln-Gln-Ala-Arg

Spheron Component peptide #5 [SEQ ID NO 5]
ALAHLLEAERQER
Ala-Leu-Ala-His-Leu-Leu-Glu-Ala-Glu-Arg-Gln-Glu-Arg

Spheron Component peptide #6 [SEQ ID NO 6]
AADHDVGSELPPEGVLGALLR
Ala-Ala-Asp-His-Asp-Val-Gly-Ser-Glu-Leu-Pro-Pro-Glu-Gly
Val-Leu-Gly-Ala-Leu-Leu-Arg

Spheron Component peptide #7 [SEQ ID NO 7]
LETPAPQVPAR
Leu-Glu-Thr-Pro-Ala-Pro-Gln-Val-Pro-Ala-Arg

Spheron Component peptide #8 [SEQ ID NO 8]
ILAGSADSEGVAAPR
Ile-Leu-Ala-Gly-Ser-Ala-Asp-Ser-Glu-Gly-Val-Ala-Ala-Pro-Arg

Spheron Component peptide #9 [SEQ ID NO 9]
ARPVKEPRGLSAASPPLAETGAPRRF
Ala-Arg-Pro-Val-Lys-Glu-Pro-Gly-Leu-Ser-Ala-Ala-Ser-Pro-Pro-Leu-Ala-Glu-Thr-Gly-Ala-Pro-Arg-Arg-Phe

Spheron Component peptide #10 [SEQ ID NO 10]
ARPVKEP
Ala-Arg-Pro-Val-Lys-Glu-Pro

Spheron Component peptide #11 [SEQ ID NO 11]
GLSAASPPLAETGAPRRF
Gly-Leu-Ser-Ala-Ala-Ser-Pro-Pro-Leu-Ala-Glu-Thr-Gly-Ala-Pro-Arg-Arg-Phe

Spheron Component peptide #12 [SEQ ID NO 12]
AADHDVGSELPPEGVLGALLRVKRLETPAPQVPA
Ala-Ala-Asp-His-Asp-Val-Gly-Ser-Glu-Leu-Pro-Pro-Glu-Gly-Val-Leu-Gly-Ala-Leu-Leu-Arg-Val-Lys-Arg-Leu-Glu-Thr-Pro-Ala-Pro-Gln-Val-Pro-Ala

Spheron Component peptide #13 [SEQ ID NO 13]
AADHDVGSELPPEGVLGALLRV
Ala-Ala-Asp-His-Asp-Val-Gly-Ser-Glu-Leu-Pro-Pro-Glu-Gly-Val-Leu-Gly-Ala-Leu-Leu-Arg-Val

Spheron Component peptide #14 [SEQ ID NO 14]
LETPAPQVPA
Leu-Glu-Thr-Pro-Ala-Pro-Gln-Val-Pro-Ala

Spheron Component peptide #15 [SEQ ID NO 15]
RRSVPRGEAAG
Arg-Arg-Ser-Val-Pro-Arg-Gly-Glu-Ala-Ala-Gly

Spheron Component peptide #16 [SEQ ID NO 16]
VLAQLLRVWGAPRNSD
Val-Leu-Ala-Gln-Leu-Leu-Arg-Val-Trp-Gly-Ala-Pro-Arg-Asn-Ser-Asp

Spheron Component peptide #17 [SEQ ID NO 17]
PALGLDDDPDAPAAQLAR
Pro-Ala-Leu-Gly-Leu-Asp-Asp-Asp-Pro-Asp-Ala-Pro-Ala-Ala-Gln-Leu-Ala-Arg

Spheron Component peptide #18 [SEQ ID NO 18]
LARALLRARLDPAALAA
Leu-Ala-Arg-Ala-Leu-Leu-Arg-Ala-Arg-Leu-Asp-Pro-Ala-Ala-Leu-Ala-Ala

Spheron Component peptide #19 [SEQ ID NO 19]
QLVPAPVPAAALRPRPPVYDD
Gln-Leu-Val-Pro-Ala-Pro-Val-Pro-Ala-ala-Ala-Leu-Arg-Pro-Arg-Pro-Pro-Val-Tyr-Asp-Asp

Spheron Component peptide #20 [SEQ ID NO 20]
GPAGPDAEEAGDE
Gly-Pro-Ala-Gly-Pro-Asp-Ala-Glu-Glu-Ala-Gly-Asp-Glu

Spheron Component peptide #21 [SEQ ID NO 21]
TPDVDPELLRYLLGR
Thr-Pro-Asp-Val-Asp-Pro-Glu-Leu-Leu-Arg-Tyr-Leu-Leu-Gly-Arg

Spheron Component peptide #22 [SEQ ID NO 22]
LLRVKR
Leu-Leu-Arg-Val-Lys-Arg

The afore-mentioned spheron component peptides can be used to identify compounds or combinations and mixtures thereof that are useful in preventing or reducing the effects of these peptides in the brain. Preventing or reducing the effects of these spheron component peptides will serve to treat and/or ameliorate symptoms that are believed to be the same symptoms associated with Alzheimer's Disease, but which are not brought about by amyloid plaque formation. Preventing or reducing the effects of these spheron component peptides also will serve to treat and/ or ameliorate many other symptoms of Alzheimer's Disease and other disorders brought about by the presence of these spheron components in such high concentrations in the brain at or near the loci of the spherons.

One aspect of the invention entails a method of identifying compounds useful in treating and/or ameliorating the above-described symptoms by culturing reproducible cells, preferably tumor-forming cells, more preferably, glioma and neuroblastoma cells, adding at least one spheron component peptide to the cell culture, and then adding a test compound to some of the cell cultures. The method then entails measuring the percentage of viable cells, and then comparing the percentage of viable cells found with the cell cultures to which test compounds were added with the percentage of viable cells found in control cell cultures to which no test compounds were added. Useful test compounds are identified as those compounds used in the afore-mentioned example, whereby the cultures have a statistically significant greater number of viable cells than the control (e.g., no compound administered).

Another method of identifying useful compounds is to culture reproducible cells that are capable of expressing one or more of the cerebral components selected from cerebral ACTH, cerebral insulin, cerebral enkephalins, cerebral TRH, cerebral dynorphin, cerebral_MSH and _endorphin, cerebral pro-insulin, cerebral pro-glucagon, cerebral pro-glucagon-like peptide, cerebral pro-somatostatin, cerebral pro-pancreatic peptide, cerebral pro-GHRH, cerebral neuropeptide melamin-concentrating hormone, cerebral NEI, cerebral neurotensin, and cerebral opioid peptides, and then adding at least one spheron component peptide to the cell culture. Test compounds then can be added to the cell culture and the amounts of the respective cerebral components listed above can be measured. Useful test compounds are identified as those compounds used in the afore-mentioned example, whereby the cultures to which they were added had increased cerebral ACTH, cerebral insulin, cerebral enkephalins, cerebral TRH, cerebral dynorphin, cerebral_MSH and _endorphin, cerebral pro-insulin, cerebral pro-glucagon, cerebral pro-somatostatin, cerebral pro-pancreatic peptide, cerebral pro-GHRH, cerebral neuropeptide melamin-concentrating hormone, cerebral NEI, cerebral neurotensin, and cerebral opioid peptides, when compared to control cultures to which no test compounds were added.

The useful test compounds also will be those compounds that: (i) impede or prevent the release by a spheron of proSAAS or peptide fragments thereof; (ii) bind to, inhibit, antagonize, or compete with proSAAS or peptide fragments thereof that are released by a spheron; or (iii) reduce or prevent the cytotoxic effects of proSAAS or peptide fragments thereof that are released by a spheron. Those skilled in the art will be capable of utilizing the isolates spheron components described herein to assay for useful compounds, using the guidelines provided herein.

Various embodiments of the invention also relate to a method of identifying compounds useful in treating or ameliorating a neurological condition caused by spheron component peptides that includes culturing reproducible cells, and then transforming, transfecting, infecting, or otherwise inducing the cultured, reproducible cells to express proSAAS or a peptide fragment, variant, derivative, homologue, or mimetic thereof. The method then includes administering to the cultured cells a test compound, and then determining whether the test compound ameliorated, eliminated, or reduced the effects of the proSAAS or a peptide fragment, variant, derivative, homologue, or mimetic thereof, when compared to controls.

Another aspect of the present invention includes a composition comprising at least one compound capable of preventing or reducing the effects of spheron component peptides. The composition can include at least one isolated polypeptide or other compound that binds to, antagonizes or competes with a spheron component peptide other than a spheron component responsible for forming amyloid plaque. These isolated polypeptides or other compound can be identified using the testing protocol described briefly above, and in more detail below. Those skilled in the art are capable of manufacturing and isolating polypeptides or other compounds that bind to, antagonize, or compete with the above-described spheron component, using the guidelines provided herein.

This invention also includes polypeptides that are antibodies to the spheron component peptides, of the present invention. Those skilled in the art are capable of generating polyclonal and monoclonal antibodies that bind to the spheron component peptides. Those skilled in the art are capable of using such antibodies to create antibody fragments, including short chain antibodies, and chimeric or humanized antibodies which have similar or better binding characteristics but have more favorable pharmacological properties such as reduced antigenicity, better bioavailability, easier manufacture and better blood-brain barrier penetration.

The composition also preferably includes at least one isolated polypeptide or compound that impedes or prevents the release from a spheron proSAAS or a peptide fragment thereof other than a spheron component responsible for forming amyloid plaque. These isolated polypeptides or compounds can be identified using the testing protocol described briefly above, and in more detail below. In addition, the compositions of the invention preferably include at least one isolated polypeptide or compound that reduces or prevents the cytotoxic or other deleterious effects of a spheron component peptide other than a spheron component responsible for forming amyloid plaque. These isolated polypeptides can be identified using the testing protocol described briefly above, and in more detail below.

The composition can be administered in a therapeutically effective amount to an animal in need thereof, e.g., an animal having at least one spheron component peptide in its neuronal gray matter in a cytotoxic amount. The method of administering the composition according to the invention includes, but is not limited to, administering the composition intramuscularly, orally, intravenously, intratumorally, intrathecally, intranasally, topically, transdermally, via an aerosol, etc.

Solid dosage forms for oral administration include capsules, tablets, pills, powders, and granules. In such solid dosage forms, the active compound is admixed with at least one of the following: (a) one or more inert excipients (or carrier), such as sodium citrate or dicalcium phosphate; (b) fillers or extenders, such as starches, lactose, sucrose, glucose, mannitol, and silicic acid; (c) binders, such as carboxymethylcellulose, alginates, gelatin, polyvinylpyrrolidone, sucrose and acacia; (d) humectants, such as glycerol; (e) disintegrating agents, such as agar-agar, calcium carbonate, potato or tapioca starch, alginic acid, certain complex silicates, and sodium carbonate; (f) solution retarders, such as paraffin; (g) absorption accelerators, such as quaternary ammonium compounds; (h) wetting agents, such as acetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Liquid dosage forms for oral administration include pharmaceutically acceptable emulsions, solutions, suspensions, syrups, and elixirs. In addition to the active compounds, the liquid dosage forms may comprise inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances, and the like.

Besides such inert diluents, the composition can also include adjuvants, such as wetting agents, emulsifying and suspending agents, sweetening, flavoring, and perfuming agents.

Another method of administering the compositions of the invention is by a transdermal or transcutaneous route. One example of such an embodiment is the use of a patch. In particular, a patch can be prepared with a fine suspension of the composition in, for example, dimethylsulfoxide (DMSO), or a mixture of DMSO with cottonseed oil and brought into contact with the skin of the tumor carrying mammals away from the tumor location site inside a skin pouch. Other mediums or mixtures thereof with other solvents and solid supports would work equally as well. The patch can contain one or more of the test compounds described above in the form of a solution or a suspension. The patch can then be applied to the skin of the patient, for example, by means of inserting it into a skin pouch of the patient formed by folding and holding the skin together by means of stitches, clips or other holding devices. This pouch should be employed in such a manner so that continuous contact with the skin is assured without the interference of the mammal. Besides using a skin pouch, any device can be used which ensures the firm placement of the patch in contact with the skin. For instance, an adhesive bandage could be used to hold the patch in place on the skin.

Actual dosage levels of active ingredients in the compositions of the invention may be varied to obtain an amount of one or more test compounds that is effective to obtain a desired therapeutic response for a particular composition and method of administration. The selected dosage level therefore depends upon the desired therapeutic effect, the route of administration, the desired duration of treatment, the size of the animal being treated, and other factors.

With mammals, including humans, the effective amounts can be administered on the basis of body surface area. The interrelationship of dosages for animals of various sizes, species, and humans (based on mg/M² of body surface) is described by E. J. Freireich et al., *Cancer Chemother*. *Rep*., 50(4):219 (1966). Body surface area may be approximately determined from the height and weight of an individual *(see e.g.,* Scientific Tables, Geigy Pharmaceuticals, Ardsley, N.Y. pp. 537-538 (1970)).

The total daily dose of composition to a host may be in single or divided doses. Dosage unit compositions may contain such amounts of such submultiples thereof as may be used to make up the daily dose. It will be understood, however, that the specific dose level for any particular patient will depend upon a variety of factors including the body weight, general health, sex, diet, time and route of administration, potency of the administered drug, rates of absorption and excretion, combination with other drugs and the severity of the particular disease being treated.

Because the composition is targeted to prevent unwanted cytoxiticity and other deleterious effects in the brain, the method of administration can encompass formulating a composition to further contain an agent that enables the test compound to cross the blood-brain barrier. Such methods of administration are described in, for example, U.S. Patent Nos. 5,008,257, 4,824,850, 4,479,932, 4,727,079, 4,622,218, and 4,540,564.

The treatment of nervous system disorders or other brain-related disorders can be achieved by administering drugs that affect nervous system function or dysfunction in animals or patients. Typically, such drugs are administered by peripheral application, either via the oral or the systemic route. While some drugs are able to cross the blood brain barrier (bbb), others do not pass the bbb efficiently or not at all and are only effective when given directly into the brain. The term "blood-brain barrier" or "bbb", as used herein, refers to the bbb proper as well as to the blood-spinal barrier. The blood-brain barrier, which consists of the endothelium of the brain vessels, the basal membrane and neuroglial cells, acts to limit penetration of substances into the brain. Sometimes the structure of the bbb is subdivided into two components: the endothelial or capillary barrier and the ependymal barrier. Banks, W. A., Kastin, A. J., Barrera, "Delivering peptides to the central nervous system: Dilemmas and strategies," *Pharm. Res.* 8:1345-1350 (1991).

The nature of the substance penetration through the bbb has not yet been determined but it is known that many of the regulators of brain function such as cytokines, transferrin, encephalins and endorphines can pass through the bbb from the blood vessels into the brain Raeissi, S., Audus, J., *"In vitro* characterization of blood-brain barrier permeability to delta sleep-inducing peptide." J. *Pharm. Phy.* 41:848-852(1989); Zlokovich, B., Susie, V. T., Davson, H. Begley, D. J., Jankov, R. M., Mitrivic, B. M., Lipovac, M. N., "Saturable mechanism for delta sleep-inducing peptide (DSIP) at the blood-brain barrier of the vascularly perfused guinea pig brain." *Peptides* 10:249-254(1989); and Zlokovich, B., *"In vivo* approaches for studying peptide interaction at the blood-brain barrier." J. *Control. Rel*. 13:185-201(1990). However, many substances that can affect the Central Nervous System (or CNS) such as adenosine, β-endorphin, synthetic analogs of endogenous peptides Houghten, R. A. Swann, R. W., Li, C. H., "β-Endorphin: Stability, clearance behaviour and entry into the central nervous system after intravenous injection of the tritiated peptide in rats and rabbits." *Proc. Natl. Acad. Sci. USA* 77:4588-4591(1980); Levin, E. R., Frank, H. J. K., Weber, M. A., Ismail, M., Mills M., "Studies on penetration of the blood-brain barrier by atrial natriuretic factor." *Biochem. Biophys. Res. Commun.* 147:1226-1231(1987) Sakane, T., Tanaka, C., Yamamoto, A., Hashida, M., Sesaki, H., Ueda, H., Takagi, H., "The effect of polysorbate 80 on brain uptake and analgesic effect of D-kyoto." *Int.* J. *Pharm.* 57:77-83(1989), as well as some excitatory and inhibitor amino acids and trophic factors, penetrate poorly or not at all through the bbb. At present, drugs with no bbb penetration or poor bbb penetration can only be given by direct CNS infusion or by implantation of controlled-release polymers. *(See,* e.g., U.S. Pat. No. 4,883,666, Sabel *et al.).*

One way to overcome some of the limitations of traditional drug therapy is to increase the relative amount of drug that passes the bbb. The belief is that if one can increase the amount of the drug crossing the bbb while reducing the peripheral dose of a given drug or diagnostic substance, the peripheral side effects of the drug are also less severe, while at the same time maintaining the desired effect in the brain. A number of approaches have been described as effective in increasing drug penetration through the bbb.

One approach has been to alter the function of the bbb itself. For instance, osmotic agents, when given peripherally (such as by intravenous injection), result in the opening of the bbb. Further, some drugs acting on the CNS can change the permeability of the bbb for other substances; cholinomimetic arecolines, for instance, have been reported to induce changes of drug penetration through the bbb Saija, A., Princi, P., De Pasquale, R., Costa, G., "Arecoline but not haloperidol produces changes in the permeability of the blood-brain barrier in the rat." *J. Pharm. Pha.* 42:135-138 (1990).

Other drugs that can be administered to alter the permeability of the bbb are disclosed in U.S. Pat. Nos. 5,059,415 and 5,124,146, both issued to E. A. Neuwelt. Bradykinin is one specific drug with such effects. (U.S. Pat. No. 5,112,596, issued to Malfroy-Camine). Another method comprises administering permeabilizer peptides such as A-7 or conformational analogs thereof. (WO 92/18529, an application of J. W. Kozarich et al.). A relatively invasive method has been proposed by A. Tomasz and E. Tuomanen (WO 91/16064) who administer parenteral injections of purified cell wall or cell wall fragments of eubacteria such as *Streptococcus pneumoniae* to open the bbb.

U.S. Pat. No. 5,260,210 issued to L. L. Rubin *et al.,* discloses a method whereby the permeability of the blood-brain barrier is increased by administering an agent that reduces or interferes with cyclic AMP concentrations or that increases cyclic GMP concentrations.

Another approach is the modification of the drug molecules themselves. For instance, macromolecules, such as proteins, do not pass the bbb at all, or pass through with difficulty or with alterations that adversely impact the proteins efficacy. For example, one can first isolate the macromolecule active site, i.e., the portion of the molecule that triggers the biologically desirable event, and then use only this active site. Since size is one of the factors in allowing permeability of the bbb, the reduced size can be used so that the smaller molecule can now pass the bbb. Other modifications to macromolecules to attempt passage of the bbb include glycating the proteins, thereby enhancing their permeability of the bbb, or forming a prodrug. U.S. Pat. No. 5,260,308, issued to J. F. Podusio and G. L. Curran, discusses glycating proteins, while U.S. Pat. No. 4,933,324 and WO 89/07938, both on applications of V. E. Shashoua, disclose formation of a prodrug. These prodrugs are formed from a fatty acid carrier and a neuroactive drug which is unable to pass across the bbb on its own. A similar system is disclosed in WO 89/07938.

Still another approach is the implantation of controlled release polymers that release the active ingredient from a matrix-system directly into the nervous tissue. However, this approach is invasive and requires surgical intervention if implanted directly into the brain or spinal cord (see Sabel et *al.* U.S. Pat. No. 4,883,666; and Sabel *et al.* U.S. patent application Ser. No. 07/407,930). It also is known to administer compositions directly to internal portions of the brain, as disclosed on U.S. Pat. No. 5,800,390, These methods enable the delivery of sustained release, solid preparations and semi-solid preparations directly to brain tissue.

To overcome these limitations, another approach has been tried in which drug carrier systems are used such as liposomes, erythrocyte ghosts, antibody-conjugates, and monoclonal antibody conjugates. One of the major problems in targeted drug delivery is the rapid opsonization and uptake of injected carriers by the reticuloendothelial system (RES), especially by the macrophages in the liver and spleen. This obstacle may be partially overcome in the case of liposomes by incorporation of so-called "stealth" lipids, such as phosphatidylinositol, monosialoganglioside, or sulfogalactosylceramide.

U.S. Pat. Nos. 5,182,107 and 5,154,924, both issued to P. M. Friden, teach a method of conjugating a drug with an antibody where the antibody is reactive with a transferrin receptor. Transferrin receptors are located on brain capillary endothelial cells, which thus can transport a drug, such as nerve growth factor, across the bbb. U.S. Pat. No. 5,004,697 (issued to Pardridge) improves such an antibody-conjugate method by providing cationized antibodies with a specific isoelectric point (see also WO 89/01343 by Pardridge).

Another approach is to create chimeric peptides to which the active agents are conjugated (U.S. Pat. No. 4,801,575, also issued to Pardridge). Such a system is further discussed also in U.S. Pat. No. 4,902,505, issued to Pardridge and Schimmel, in which the chimeric peptide, such as histone, is capable of crossing the bbb by transcytosis.

U.S. Pat. Nos. 5,187,158 and 5,017,566, both issued to N. S. Bodor, disclose a brain-specific drug delivery method wherein a centrally acting drug is given with the reduced, biooxidizable lipoidal form of a dihydropyridine reaction-pyridine salt redox carrier such as dopamine. *(See also* U.S. Pat. No. 4,880,816, also issued to Bodor).

A rather invasive approach is taken to deliver genetic material to the brain. This is done, for example, by chemically disrupting the bbb and then using viruses to deliver genes across the bbb. *(See,* U.S. Pat. No. 4,866,042, issued to E. A. Neuwelt). Here, a corrective genetic material is incorporated into a virus and the virus is then injected into the bloodstream.

Finally, yet another carrier system to deliver drugs across the bbb is the use of liposomes, as disclosed by F. D. Collins and R. C. Thompson (WO 91/04014). Here, liposomes are targeted to specific endogenous brain transport systems that transport specific ligands across the bbb.

Another approach is disclosed in U.S. Patent No. 6,117,454, to Kreuter, *et al.* The subject matter of the Kreuter patent includes a method, composition and drug targeting system using surfactant coated nanoparticles as a drug carrier (or targeting molecule) for a wide range of drugs in order to enhance the penetration of drugs or diagnostic agents across the bbb. Any of these methods can be used in the invention to administer a component, (e.g., compound, composition, peptide, gene, etc.) capable of preventing the cytotoxic effect of the spheron component peptides discussed above into the brain.

Another embodiment of the invention encompasses an Alzheimer's Disease symptom non-human animal model, a method of making the non-human animal model, and a method of using the non-human animal model to identify effective therapies for treating or ameliorating brain damage or other disorders or maladies caused by spherons releasing component peptides other than those directly responsible for forming amyloid plaque. The non-human animal model comprises an non-human animal having a gene inserted into the brain thereof, whereby the gene expresses one or more spheron component peptides selected from the group defined above. The method of making the non-human animal model therefore includes preparing a gene that over-expresses one or more of spheron component peptides selected from the group defined above, incorporating the gene into the brain of a non-human animal (e.g. by making a transgenic non-human animal, using viral vectors to insert the gene into the central nervous system, or by other methods known in the art), and inducing the gene to over-express the spheron component peptide.

The method of using the non-human animal model includes preparing a group of non-human animals having a gene or genes in the brain thereof that over-expresses one or more of spheron component peptides selected from the group defined above, and then inducing the gene to over-express the spheron component peptide. The method further includes administering to a select group of non-human animals a test compound, sacrificing the non-human animals, and then measuring the amount of isolated spheron component peptides present in the sacrificed non-human animal's brain, and/ or measuring the percentage of viable cells at or around the locus of the gene and/ or by measuring injury in relation to the protein expression, and/or measuring behavioral or other brain functional parameters. The method concludes by selecting those test compounds that reduce the amount of isolated spheron component peptides, when compared to controls, and/ or by selecting those test compounds that yield a higher percentage of viable cells at or around the locus of the gene, when compared to controls having no test compound administered thereto, and/or by selecting those test compounds that reduce injury in relation to the protein expression, and/or by selecting those test compounds that improve brain function or behavior, etc. compared to controls.

The method of using the non-human animal model to screen for effective test compounds also entails preparing the animal model as described above, and then inducing the gene to overexpress the one or more spheron component peptides selected from the group defined above. The method further includes administering to a select group of non-human animals a test compound, sacrificing the non-human animals, and then measuring the amount of one or more of the following: (i) cerebral ACTH; (ii) cerebral insulin; (iii) cerebral enkephalins; (iv) cerebral TRH; (v) cerebral dynorphin; or (vi) any of the other components descrbed previously, such as glucagon, GLP, neurotensin, and the like. The method concludes by selecting those compounds that, when administered, reveal an increased level of any one of the five components, when compared to control non-human animals to which no test compound were administered.

Introducing one or more copies of the spheron component peptides into non-human animal first entails making a gene that expresses the respective peptide(s), or that can be induced to express the peptide(s) described above. The gene then can be introduced into the brain of the non-human animal by transgenic animal techniques known in the art. The gene then will express the spheron component peptide(s), or can be induced to over-express the spheron component peptide(s). Using the guidelines provided herein, those skilled in the art are capable of making a gene that expresses one or more of the above-described peptides without undue experimentation. Means of gene delivery to a cell or tissue include direct injection of bare DNA, ballistic methods, use of a viral vector such as a retrovirus, adenovirus, adeno-associated virus, pox virus or herpes simplex virus, use of a DNA-protein conjugate and use of a liposome. Examples of using viral vectors to introduce genes into the central nervous system to treat brain disorders such as Parkinson's disease are disclosed in U.S. Patent No. 6,248,320. The spheron component peptides are those listed above as SEQ ID NOS 1-3 and 5-22

The invention also includes various cell lines that have been transfected, transformed, or infected in accordance with the methods of the invention. These cell lines preferably include recombinant cells that express a nucleic acid sequence that encodes. an amino acid sequence selected from SEQ ID Nos. 1-3, 5-14, or combinations thereof.

### EXAMPLES

### Example 1

Spherons were extracted and purified to homogeneity from human brain according to the method of U.S. patent Nos. 6,309,892, and 5,525,339. Samples of homogeneous spherons were separated by polyacrylamide gel electrophoresis according to standard methods recited in U.S. patent Nos. 6,309,892, and 5,525,339. The bands on the gels were transferred to nitrocellulose blots by standard methods well known in the art. These bands were further analyzed by high performance liquid chromatography and amino acid microsequencing analysis.

The following sequences were among those detected from purified homogeneous dense microsphere preparations:
1) GEAAGAVQELAR (SEQ ID NO. 1);
2) GLSAASPPLAETGAPR (SEQ ID NO. 2);
3) ARAEAQEAEDQQAR (SEQ ID NO. 3);
4) VLAQLLR (SEQ ID NO. 4);
5) ALAHLLEAERQER (SEQ ID NO. 5);
6) AADHDVGSELPPEGVLGALLR (SEQ ID NO. 6);
7) LETPAPQVPAR (SEQ ID NO. 7);
8) ILAGSADSEGVAAPR (SEQ ID NO. 8);
* listed for illustrative purposes only

These sequences are consistent with all or a portion of a protein referred to as pro-SAAS, and also known as granin-like neuroendocrine peptide precursor.

### Example 2

Polypeptides corresponding to spheron component peptides sequenced in Example 1 were synthesized and tested in neuronal cell cultures. The spheron component peptides in phosphate buffered saline (PBS) were incubated alone and in combination into glioma cell cultures and neuroblastoma cell cultures. Controls of
1) homogeneous samples of spherons, 0.1-10 mg protein/mL;
2) phosphate buffered normal saline alone;
3) tamoxifen, 100 µM;
4) DMSO; and
5) bovine serum albumin, 5 mg/mL.
also were tested in the cultures.

### Cell culture:

Cryopreserved glioma and neuroblastoma cells were acquired from the American Type Culture Collection (ATCC), Virginia. Cells were thawed and diluted in suspension media and centrifuged at 700 rpm for 7 minutes at 4C. Cell pellets then were resuspended in CACO-2 media and cultured in standard 75 or 175 cm² flasks at 37C, 5% CO(2) until approximately confluent. The cells were then trypsinized and resuspended in CACO-2 media to achieve a final cell density of 1.5 X 10⁵ cells/mL. 50 µL aliquots per sample were then added per well to 96 well plates.

### Dosing:

For Experiment I,50 µL of either PBS (phosphate buffered solution) (negative control), 100 µM tamoxifen in phosphate buffered saline PBS (positive control), or test article were added to each sample culture.

For Experiment II, 1mM tamoxifen in DMSO (dimethylsufoxide) was diluted to 100 µM concentration in CACO-2 media, and 100 µL per well was added to positive control wells. A vehicle control consisting of 1 % DMSO in CACO-2 media was also added to this experiment. Other negative controls consisted of human and bovine serum albumin.

### MTT assays:

An MTT assay is a sensitive assay for the measurement of cell proliferation based upon the reduction of the tetrazolium salt 3, [4,5-dimetylthiazol-2-yl] -2,5-diphenyltetrazolium bromide (MTT). Following incubations with control or test substances, media was aspirated and 100 µL MTT was added to each well. Plates were then incubated for 3 hours at 37 C, 5% CO(2). MTT was then replaced with acidified isopropanol (0.4 N HCl) and the plates were stored overnight at 4 C, covered. Plates were then agitated gently for 1 minute on a rotary shaker and the difference between emission absorbance of 570 nm and background absorbance of 650 nm was measured spectrophotometrically on an automated plate reader.

### Results:

### Experiment I:

Glioma cells were incubated for 24 hours and 96 hours following treatment with test materials or control solutions. MTT was added to all cultures at this time. Results are expressed as mean absorbance differences and represented as per cent of negative control and are shown in Table 3 and Table 4 (cytotoxicity in glioma cells).

**Table 3: Cytotoxicity in glioma cells: 24 hrs.**

| Test Article | Concentration | ABS 570-690 | | Percent of Viable Cells |
|---|---|---|---|---|
| Identification | | Sample | SD | |
| NC (PBS) | | 0.093 | 0.016 | 100 |
| VC (DMSO) | | 0.079 | 0.004 | 100 |
| PC (Tamoxifen) | 100 µM | 0.014 | 0.004 | 18 |
| Synthetic peptide | 5 mg/mL | 0.044 | 0.020 | 72 |
| Spherons | 100-1000 ng/ mL | 0.048 | 0.005 | 79 |

| | | | | |
|---|---|---|---|---|
| Abbreviations: ABS, Absorbance; NC, Negative Control; PC, Positive Control; SD, Standard Deviation; VC, Vehicle Control. | | | | |

**Table 4: Cytotoxicity in glioma cells: 4 days**

| Test Article | Concentration | ABS 570-690 | | Percent of Viable Cells |
|---|---|---|---|---|
| Identification | | Sample | SD | |
| NC (PBS) | | 0.590 | 0.048 | 100 |
| PC (Tamoxifen) | 100 µM | 0.700 | 0.052 | 63 |
| Synthetic peptides i | 5 mg/mL | 0.150 | 0.032 | 25-75 |
| Synthetic peptides ii | 5 mg/mL | 0.030 | 0.003 | 5-50 |
| Spherons | | 0.070 | 0.014 | 11-60 |

| | | | | |
|---|---|---|---|---|
| Abbreviations: ABS, Absorbance; NC, Negative Control; PC, Positive Control; SD, Standard Deviation; VC, Vehicle Control | | | | |

### Experiment II:

Neuroblastoma cells were used for this experiment. MTT was added to the plates following a 24 hour and 96 hour incubation with control or test solutions. Other plates were replenished with fresh media, control, or test solution at 24 hours and then read 3 days later. Results are expressed as mean absorbance differences and as per cent of negative control and are shown in Table 5. Similar results were found in 96 hours.

**Table 5: Neuroblastoma cells: 24 hour incubation**

| Test Article | Concentration | ABS 570-690 | | Percent of Viable Cells |
|---|---|---|---|---|
| Identification | | Sample | SD | |
| NC (PBS) | | 0.089 | 0.009 | 100 |
| VC (DMSO) | | 0.073 | 0.004 | 100 |
| PC (Tamoxifen) | 100 µM | 0.010 | 0.001 | 14 |
| Synthetic proteins | 5 mg/ mL | 0.012 | 0.003 | 13-60 |
| Synthetic proteins | 0.5 mg/mL | 0.050 | 0.011 | 52-70 |
| Synthetic proteins | 0.05 mg/mL | 0.057 | 0.004 | 59-70 |
| Spherons | | 0.059 | 0.037 | 61 |

| | | | | |
|---|---|---|---|---|
| Abbreviations: ABS, Absorbance; NC, Negative Control; PC, Positive Control; SD, Standard Deviation; VC, Vehicle Control | | | | |

### Conclusion:

Significant cytotoxic effects on glioma and neuroblastoma cells are apparent at 24 hours and in glioma cells at 96 hours with spherons and with synthetic spheron component peptides proteins from spherons.

### Example 3

The synthetic peptides of Example 2, diluted in phosphate buffered saline at 1-5 mg/mL were inoculated into the cerebral cortex of 12 normal rats. Control rats received phosphate buffered saline. The animals were observed and painlessly sacrificed after intervals of 1, 4, and 10 days. The rat brains were fixed in 10% formalin, embedded in paraffin, sectioned and stained with hematoxylin-eosin and examined by light microscopy. In all examples, acute inflammatory reactions with extreme microglial reaction were observed. This degree of gliosis and inflammation was not found in the controls.

### Example 4

The genes for spherons and genes for spheron component peptide fragments thereof are transfected into neuronal cell lines and expression of the genes is promoted. The neuronal cells are examined for evidence of abnormal cell loss and abnormal pathological changes.

### Example 5

The genes for spherons and genes for spheron component peptide fragments thereof are transfected into experimental animal brains and expression of the genes is promoted. The experimental animals are observed for abnormal behavior, and the experimental animal brains are examined for evidence of histopathological abnormalities.

## Claims

1. A method of identifying compounds useful in treating or ameliorating a neurological condition comprising:
contacting a test compound with a spheron component peptide other than a spheron component responsible for forming amyloid plaque, the spheron component peptide being selected from the group consisting of:
GEAAGAVQELAR (SEQ ID NO. 1);
GLSAASPPLAETGAPR (SEQ ID NO. 2);
ARAEAQEAEDQQAR (SEQ ID NO. 3);
ALAHLLEAERQER (SEQ ID NO. 5);
AADHDVGSELPPEGVLGALLR (SEQ ID NO. 6);
LETPAPQVPAR (SEQ ID NO. 7);
ILAGSADSEGVAAPR (SEQ ID NO. 8);
ARPVKEPRGLSAASPPLAETGAPRRF (SEQ ID NO. 9);
ARPVKEP (SEQ ID NO. 10);
GLSAASPPLAETGAPRRF (SEQ ID NO. 11);
AADHDVGSELPPEGVLGALLRVKRLETPAPQVPA (SEQ ID NO. 12);
AADHDVGSELPPEGVLGALLRV (SEQ ID NO. 13);
LETPAPQVPA (SEQ ID NO. 14);
RRSVPRGEAAG (SEQ ID NO. 15);
VLAQLLRVWGAPRNSD (SEQ ID NO. 16);
PALGLDDDPDAPAAQLAR (SEQ ID NO. 17);
LARALLRARLDPAALAA (SEQ ID NO. 18);
QLVPAPVPAAALRPRPPVYDD (SEQ ID NO. 19);
GPAGPDAEEAGDE (SEQ ID NO. 20);
TPDVDPELLRYLLGR (SEQ ID NO. 21); and
LLRVKR (SEQ ID NO. 22);
and determining whether the test compound binds to, antagonizes, or competes with the spheron component peptide,
with the proviso that if said test compound is administered to an animal that has been transfected with a transformed, transfected or infected cell comprising the spheron component peptide, said animal is a non-human animal.

2. The method of claim 1, comprising:
transforming, transfecting, or infecting cells comprising the spheron component peptide;
administering the test compound to the transformed, transfected, or infect cells; and
identifying the test compounds that ameliorate the effects of the spheron component peptide.

3. The method of claim 1 or 2, wherein the concentration of the spheron component peptide in the transformed, transfected or infected cells is sufficient to reduce the viability of the cells.

4. The method of any of claims 1-3, wherein the transformed, transfected or infected cells are cultured *in vitro.*

5. The method of any of claims 1-3, wherein the transformed, transfected or infected cells are cultured *in vivo.*

6. The method of any of claims 1-5, wherein the test compound is administered to an non-human animal that has been transfected with the transformed, transfected or infected cells.

7. The method of claim 6, further comprising:
preparing a non-human animal model by injecting into the brain of a non-human animal either a gene that expresses one or more of the spheron component peptides; and
inducing the gene to express the spheron component peptide.

8. The method of claim 7, wherein the gene is injected into the brain of the non-human animal along with a vector.

9. The method of claim 7 or 8, further comprising:
preparing a group of non-human animal models, each non-human animal model having a gene in the brain thereof that expresses one or more of the spheron component peptides;
inducing the gene if present to express the spheron component peptide;
administering a test compound to the group of non-human animal models;
sacrificing the non-human animals;
measuring the amount of isolated spheron component peptides present in the sacrificed non-human animal's brain, and/or measuring the percentage of viable cells at or around the locus of the gene; and
selecting those test compounds that reduce the amount of isolated spheron component peptides, when compared to controls, and/or by selecting those test compounds that yield a higher percentage of viable cells at or around the locus of the gene, when compared to controls having no test compound administered thereto.

10. A non-human animal model comprising a non-human animal having a gene inserted into the brain thereof, whereby the gene expresses one or more spheron component peptides other than a spheron component directly responsible for forming amyloid plaque,
wherein the spheron component peptide other than a spheron component responsible for forming amyloid plaque is selected from the group consisting of:
GEAAGAVQELAR (SEQ ID NO. 1);
GLSAASPPLAETGAPR (SEQ ID NO. 2);
ARAEAQEAEDQQAR (SEQ ID NO. 3);
ALAHLLEAERQER (SEQ ID NO. 5);
AADHDVGSELPPEGVLGALLR (SEQ ID NO. 6);
LETPAPQVPAR (SEQ ID NO. 7);
ILAGSADSEGVAAPR (SEQ ID NO. 8);
ARPVKEPRGLSAASPPLAETGAPRRF (SEQ ID NO. 9);
ARPVKEP (SEQ ID NO. 10);
GLSAASPPLAETGAPRRF (SEQ ID NO. 11);
AADHDVGSELPPEGVLGALLRVKRLETPAPQVPA (SEQ ID NO. 12);
AADHDVGSELPPEGVLGALLRV (SEQ ID NO. 13);
LETPAPQVPA (SEQ ID NO. 14);
RRSVPRGEAAG (SEQ ID NO. 15);
VLAQLLRVWGAPRNSD (SEQ ID NO. 16);
PALGLDDDPDAPAAQLAR (SEQ ID NO. 17);
LARALLRARLDPAALAA (SEQ ID NO. 18);
QLVPAPVPAAALRPRPPVYDD (SEQ ID NO. 19);
GPAGPDAEEAGDE (SEQ ID NO. 20);
TPDVDPELLRYLLGR (SEQ ID NO. 21); and
LLRVKR (SEQ ID NO. 22).

11. The non-human animal model of claim 10, produced by:
preparing a gene that expresses one or more of spheron component peptides other than a spheron component directly responsible for forming amyloid plaque; injecting the gene into the brain of the non-human animal; and
inducing the gene to express the one or more of spheron component peptides other than a spheron component directly responsible for forming amyloid plaque.

12. A transformed, transfected or infected cell line comprising recombinant cells that expresses a nucleic acid sequence that encodes a spheron component peptide other than a spheron component responsible for forming amyloid plaque, wherein the spheron component peptide other than a spheron component responsible for forming amyloid plaque is selected from the group consisting of:
GEAAGAVQELAR (SEQ ID NO. 1);
GLSAASPPLAETGAPR (SEQ ID NO. 2);
ARAEAQEAEDQQAR (SEQ ID NO. 3);
ALAHLLEAERQER (SEQ ID NO. 5);
AADHDVGSELPPEGVLGALLR (SEQ ID NO. 6);
LETPAPQVPAR (SEQ ID NO. 7);
ILAGSADSEGVAAPR (SEQ ID NO. 8);
ARPVKEPRGLSAASPPLAETGAPRRF (SEQ ID NO. 9);
ARPVKEP (SEQ ID NO. 10);
GLSAASPPLAETGAPRRF (SEQ ID NO. 11);
AADHDVGSELPPEGVLGALLRVKRLETPAPQVPA (SEQ ID NO. 12);
AADHDVGSELPPEGVLGALLRV (SEQ ID NO. 13);
LETPAPQVPA (SEQ ID NO. 14);
RRSVPRGEAAG (SEQ ID NO. 15);
VLAQLLRVWGAPRNSD (SEQ ID NO. 16);
PALGLDDDPDAPAAQLAR (SEQ ID NO. 17);
LARALLRARLDPAALAA (SEQ ID NO. 18);
QLVPAPVPAAALRPRPPVYDD (SEQ ID NO. 19);
GPAGPDAEEAGDE (SEQ ID NO. 20);
TPDVDPELLRYLLGR (SEQ ID NO. 21); and
LLRVKR (SEQ ID NO. 22).

13. The cell line of claim 12, wherein the nucleic acid sequence comprises at least one polynucleotide that encodes any of SEQ ID NOS: 1-3, and 5-22.

14. The cell line of claim 13, wherein the nucleic acid sequence comprises at least one polynucleotide that encodes any of SEQ ID NOS: 1-3, and 5-9.

15. The non-human animal model of any of claim 12-14, wherein the spheron component peptide other than a spheron component responsible for forming amyloid plaque is selected from the group consisting of SEQ ID NOS: 1-3, and 5-9.

16. A composition comprising at least one antibody, antibody fragment or short antibody chain which binds to a spheron component peptide selected from the group consisting of:
GEAAGAVQELAR (SEQ ID NO. 1);
GLSAASPPLAETGAPR (SEQ ID NO. 2);
ARAEAQEAEDQQAR (SEQ ID NO. 3);
ALAHLLEAERQER (SEQ ID NO. 5);
AADHDVGSELPPEGVLGALLR (SEQ ID NO. 6);
LETPAPQVPAR (SEQ ID NO. 7);
ILAGSADSEGVAAPR (SEQ ID NO. 8);
ARPVKEPRGLSAASPPLAETGAPRRF (SEQ ID NO. 9);
ARPVKEP (SEQ ID NO. 10);
GLSAASPPLAETGAPRRF (SEQ ID NO. 11);
AADHDVGSELPPEGVLGALLRVKRLETPAPQVPA (SEQ ID NO. 12);
AADHDVGSELPPEGVLGALLRV (SEQ ID NO. 13);
LETPAPQVPA (SEQ ID NO. 14);
RRSVPRGEAAG (SEQ ID NO. 15);
VLAQLLRVWGAPRNSD (SEQ ID NO. 16);
PALGLDDDPDAPAAQLAR (SEQ ID NO. 17);
LARALLRARLDPAALAA (SEQ ID NO. 18);
QLVPAPVPAAALRPRPPVYDD (SEQ ID NO. 19);
GPAGPDAEEAGDE (SEQ ID NO. 20);
TPDVDPELLRYLLGR (SEQ ID NO. 21); and
LLRVKR (SEQ ID NO. 22);
and a pharmaceutically acceptable buffer, diluent, adjuvant, carrier or combination thereof.

17. The composition of claim 16 for use as a medicament.

## Patentansprüche

1. Verfahren zur Identifizierung von Verbindungen, die zur Behandlung oder Verbesserung eines neurologischen Zustands geeignet sind, umfassend:
Kontaktieren einer Testverbindung mit einem anderen Sphärenkomponentenpeptid als eine Sphärenkomponente, die für die Bildung von Amyloidplaque verantwortlich ist, wobei das Sphärenkomponentenpeptid aus der Gruppe bestehend aus:
GEAAGAVQELAR (SEQ ID NO. 1);
GLSAASPPLAETGAPR (SEQ ID NO. 2);
ARAEAQEAEDQQAR (SEQ ID NO. 3);
ALAHLLEAERQER (SEQ ID NO. 5);
AADHDVGSELPPEGVLGALLR (SEQ ID NO. 6);
LETPAPQVPAR (SEQ ID NO. 7);
ILAGSADSEGVAAPR (SEQ ID NO. 8);
ARPVKEPRGLSAASPPLAETGAPRRF (SEQ ID NO. 9);
ARPVKEP (SEQ ID NO. 10);
GLSAASPPLAETGAPRRF (SEQ ID NO. 11);
AADHDVGSELPPEGVLGALLRVKRLETPAPQVPA (SEQ ID NO. 12);
AADHDVGSELPPEGVLGALLRV (SEQ ID NO. 13);
LETPAPQVPA (SEQ ID NO. 14);
RRSVPRGEAAG (SEQ ID NO. 15);
VLAQLLRVWGAPRNSD (SEQ ID NO. 16);
PALGLDDDPDAPAAQLAR (SEQ ID NO. 17);
LARALLRARLDPAALAA (SEQ ID NO. 18);
QLVPAPVPAAALRPRPPVYDD (SEQ ID NO. 19);
GPAGPDAEEAGDE (SEQ ID NO. 20);
TPDVDPELLRYLLGR (SEQ ID NO. 21); und
LLRVKR (SEQ ID NO. 22),
ausgewählt ist;
und Ermittlung, inwieweit die Testverbindung an das Sphärenkomponentenpeptid bindet, sie antagonisiert oder mit ihr konkurriert,
mit der Maßgabe, dass, wenn die Testverbindung einem Tier verabreicht wird, das mit einer transformierten, transfizierten oder infizierten Zelle, die das Sphärenkomponentenpeptid umfasst, transfiziert worden ist, das Tier ein nicht-humanes Tier ist.

2. Verfahren nach Anspruch 1, umfassend:
Transformierung, Transfizierung oder Infizierung von Zellen, die das Sphärenkomponentenpeptid umfassen;
Verabreichung der Testverbindung an die transformierten, transfizierten oder infizierten Zellen; und
Identifizierung der Testverbindungen, die die Wirkungen des Sphärenkomponentenpeptids verbessern.

3. Verfahren nach Anspruch 1 oder 2, worin die Konzentration des Sphärenkomponentenpeptids in den transformierten, transfizierten oder Infizierten Zellen ausreichend ist, um die Lebensfähigkeit der Zellen zu reduzieren.

4. Verfahren nach irgendeinem der Ansprüche 1-3, worin die transformierten, transfizierten oder Infizierten Zellen *in vitro* gezüchtet sind.

5. Verfahren nach irgendeinem der Ansprüche 1-3, worin die transformierten, transfizierten oder infizierten Zellen *in vivo* gezüchtet sind.

6. Verfahren nach irgendeinem der Ansprüche 1-5, worin die Testverbindung einem nicht-humanen Tier verabreicht wird, das mit den transformierten, transfizierten oder Infizierten Zellen transfiziert worden ist.

7. Verfahren nach Anspruch 6, weiterhin umfassend:
Herstellung eines nicht-humanen Tiermodells durch Injektion eines Gens, das ein oder mehrere der Sphärenkomponentenpeptide ausdrückt, in das Gehirn eines nicht-humanen Tiers; und
Induzieren des Gens, um das Sphärenkomponentenpeptid auszudrücken.

8. Verfahren nach Anspruch 7, worin das Gen zusammen mit einem Vektor in das Gehirn des nicht-humanen Tiers injiziert wird.

9. Verfahren nach Anspruch 7 oder 8, weiterhin umfassend:
Herstellen einer Gruppe von nicht-humanen Tiermodellen, wobei jedes nichthumane Tiermodell in seinem Gehirn ein Gen besitzt, das ein oder mehrere der Sphärenkomponentenpeptide ausdrückt;
Induzieren des eventuell vorhandenen Gens, um das Sphärenkomponentenpeptid auszudrücken;
Verabreichen einer Testverbindung an die Gruppe von nicht-humanen Tiermodellen;
Töten der nicht-humanen Tiere;
Messen der Menge isolierter Sphärenkomponentenpeptide, die im Gehirn des getöteten nicht-humanen Tiers vorkommen, und/oder Messung des Prozentsatzes von lebensfähigen Zellen am Genort oder um den Genort herum; und
Auswählen der Testverbindungen, die beim Vergleich mit Kontrollen die Menge isolierter Sphärenkomponentenpeptide reduzieren, und/oder Auswahl der Testverbindungen, die beim Vergleich mit Kontrollen, denen keine Testverbindung verabreicht wird, einen höheren Prozentsatz von lebensfähigen Zellen am Genort oder um den Genort herum erzielen.

10. Nicht-humanes Tiermodell, das ein nicht-humanes Tier umfasst, in dessen Gehirn ein Gen eingeführt wird, wobei das Gen ein oder mehrere andere Sphärenkomponentenpeptide als eine Sphärenkomponente, die für die Bildung von Amyloidplaque unmittelbar verantwortlich ist, ausdrückt
worin das andere Sphärenkomponentenpeptid als eine Sphärenkomponente, die für die Bildung von Amyloidplaque verantwortlich ist, aus der Gruppe bestehend aus:
GEAAGAVQELAR (SEQ ID NO. 1);
GLSAASPPLAETGAPR (SEQ ID NO. 2);
ARAEAQEAEDQQAR (SEQ ID NO. 3);
ALAHLLEAERQER (SEQ ID NO. 5);
AADHDVGSELPPEGVLGALLR (SEQ ID NO. 6);
LETPAPQVPAR (SEQ ID NO. 7);
ILAGSADSEGVAAPR (SEQ ID NO. 8);
ARPVKEPRGLSAASPPLAETGAPRRF (SEQ ID NO. 9);
ARPVKEP (SEQ ID NO. 10);
GLSAASPPLAETGAPRRF (SEQ ID NO. 11);
AADHDVGSELPPEGVLGALLRVKRLETPAPQVPA (SEQ ID NO. 12);
AADHDVGSELPPEGVLGALLRV (SEQ ID NO. 13);
LETPAPQVPA (SEQ ID NO. 14);
RRSVPRGEAAG (SEQ ID NO. 15);
VLAQLLRVWGAPRNSD (SEQ ID NO. 16);
PALGLDDDPDAPAAQLAR (SEQ ID NO. 17);
LARALLRARLDPAALAA (SEQ ID NO. 18);
QLVPAPVPAAALRPRPPVYDD (SEQ ID NO. 19);
GPAGPDAEEAGDE (SEQ ID NO. 20);
TPDVDPELLRYLLGR (SEQ ID NO. 21); und
LLRVKR (SEQ ID NO. 22),
ausgewählt ist.

11. Nicht-humanes Tiermodell nach Anspruch 10, hervorgebracht durch:
Herstellen eines Gens, das ein oder mehrere andere Sphärenkomponentenpeptide als eine Sphärenkomponente, die für die Bildung von Amyloidplaque unmittelbar verantwortlich ist, ausdrückt;
Injizieren des Gens in das Gehirn des nicht-humanen Tiers; und
Induzieren des Gens, um das eine oder mehrere andere Sphärenkomponentenpeptide als eine Sphärenkomponente, die für die Bildung von Amyloidplaque unmittelbar verantwortlich ist, auszudrücken;

12. Transformierte, transfizierte oder infizierte Zelllinie, umfassend rekombinante Zellen, die eine Nukleinsäuresequenz ausdrückt, die für ein anderes Sphärenkomponentenpeptid als eine Sphärenkomponente, die für die Bildung von Amyloidplaque verantwortlich ist, kodiert, worin das andere Sphärenkomponentenpeptid als eine Sphärenkomponente, die für die Bildung von Amyloidplaque unmittelbar verantwortlich ist, aus der Gruppe bestehend aus:
GEAAGAVQELAR (SEQ ID NO. 1);
GLSAASPPLAETGAPR (SEQ ID NO. 2);
ARAEAQEAEDQQAR (SEQ ID NO. 3);
ALAHLLEAERQER (SEQ ID NO. 5);
AADHDVGSELPPEGVLGALLR (SEQ ID NO. 6);
LETPAPQVPAR (SEQ ID NO. 7);
ILAGSADSEGVAAPR (SEQ ID NO. 8);
ARPVKEPRGLSAASPPLAETGAPRRF (SEQ ID NO. 9);
ARPVKEP (SEQ ID NO. 10);
GLSAASPPLAETGAPRRF (SEQ ID NO. 11);
AADHDVGSELPPEGVLGALLRVKRLETPAPQVPA (SEQ ID NO. 12);
AADHDVGSELPPEGVLGALLRV (SEQ ID NO. 13);
LETPAPQVPA (SEQ ID NO. 14);
RRSVPRGEAAG (SEQ ID NO. 15);
VLAQLLRVWGAPRNSD (SEQ ID NO. 16);
PALGLDDDPDAPAAQLAR (SEQ ID NO. 17);
LARALLRARLDPAALAA (SEQ ID NO. 18);
QLVPAPVPAAALRPRPPVYDD (SEQ ID NO. 19);
GPAGPDAEEAGDE (SEQ ID NO. 20);
TPDVDPELLRYLLGR (SEQ ID NO. 21); und
LLRVKR (SEQ ID NO. 22),
ausgewählt ist.

13. Zelllinie nach Anspruch 12, worin die Nukleinsäuresequenz mindestens ein Polynukleotid umfasst, das für irgendeins von SEQ ID NOS: 1-3, und 5-22 kodiert.

14. Zelllinie nach Anspruch 13, worin die Nukleinsäuresequenz mindestens ein Polynukleotid umfasst, das für irgendeins von SEQ ID NOS: 1-3, und 5-9 kodiert.

15. Nicht-humanes Tiermodell nach Irgendeinem der Ansprüche 12-14, worin das andere Sphärenkomponentenpeptid als eine Sphärenkomponente, die für die Bildung von Amyloidplaque verantwortlich ist, aus der Gruppe bestehend aus SEQ ID NOS: 1-3, und 5-9 ausgewählt ist.

16. Zusammensetzung, umfassend mindestens einen Antikörper, ein Antikörperfragment oder eine kurze Antikörperkette, der/das/die an ein Sphärenkomponentenpeptid bindet, das aus der Gruppe bestehend aus:
GEAAGAVQELAR (SEQ ID NO. 1);
GLSAASPPLAETGAPR (SEQ ID NO. 2);
ARAEAQEAEDQQAR (SEQ ID NO. 3);
ALAHLLEAERQER (SEQ ID NO. 5);
AADHDVGSELPPEGVLGALLR (SEQ ID NO. 6);
LETPAPQVPAR (SEQ ID NO. 7);
ILAGSADSEGVAAPR (SEQ ID NO. 8);
ARPVKEPRGLSAASPPLAETGAPRRF (SEQ ID NO. 9);
ARPVKEP (SEQ ID NO. 10);
GLSAASPPLAETGAPRRF (SEQ ID NO. 11);
AADHDVGSELPPEGVLGALLRVKRLETPAPQVPA (SEQ ID NO. 12);
AADHDVGSELPPEGVLGALLRV (SEQ ID NO. 13);
LETPAPQVPA (SEQ ID NO. 14);
RRSVPRGEAAG (SEQ ID NO. 15);
VLAQLLRVWGAPRNSD (SEQ ID NO. 16);
PALGLDDDPDAPAAQLAR (SEQ ID NO. 17);
LARALLRARLDPAALAA (SEQ ID NO. 18);
QLVPAPVPAAALRPRPPVYDD (SEQ ID NO. 19);
GPAGPDAEEAGDE (SEQ ID NO. 20);
TPDVDPELLRYLLGR (SEQ ID NO. 21); und
LLRVKR (SEQ ID NO. 22),
ausgewählt ist;
und einen pharmazeutisch akzeptablen Puffer, Verdünnungsmittel, Zusatz, Träger oder eine Kombination hiervon.

17. Zusammensetzung nach Anspruch 16 zur Anwendung als ein Arzneimittel.

## Revendications

1. Procédé d'identification de composés utiles dans le traitement ou l'amélioration d'une condition neurologique comprenant:
mettre en contact un composé d'essai avec un peptide à composant de sphéron autre que le composant de sphéron responsable de la formation d'une plaque amyloïde, ledit peptide à composant de sphéron étant choisi dans le groupe constitué de:
GEAAGAVQELAR (SEQ ID NO. 1);
GLSAASPPLAETGAPR (SEQ ID NO. 2);
ARAEAQEAEDQQAR (SEQ ID NO. 3);
ALAHLLEAERQER (SEQ ID NO. 5);
AADHDVGSELPPEGVLGALLR (SEQ ID NO. 6);
LETPAPQVPAR (SEQ ID NO. 7);
ILAGSADSEGVAAPR (SEQ ID NO. 8);
ARPVKEPRGLSAASPPLAETGAPRRF (SEQ ID NO. 9);
ARPVKEP (SEQ ID NO. 10);
GLSAASPPLAETGAPRRF (SEQ ID NO. 11);
AADHDVGSELPPEGVLGALLRVKRLETPAPQVPA (SEQ ID NO. 12);
AADHDVGSELPPEGVLGALLRV (SEQ ID NO. 13);
LETPAPQVPA (SEQ ID NO. 14);
RRSVPRGEAAG (SEQ ID NO. 15);
VLAQLLRVWGAPRNSD (SEQ ID NO. 16);
PALGLDDDPDAPAAQLAR (SEQ ID NO. 17);
LARALLRARLDPAALAA (SEQ ID NO. 18);
QLVPAPVPAAALRPRPPVYDD (SEQ ID NO. 19);
GPAGPDAEEAGDE (SEQ ID NO. 20);
TPDVDPELLRYLLGR (SEQ ID NO. 21); et
LLRVKR (SEQ ID NO. 22);
et déterminer si le composé d'essai se fixe à, antagonise ou se trouve en concurrence avec le peptide à composant de sphéron,
sous réserve que lorsque le composé d'essai est administré à un animal ayant été transfecté par une cellule transformée, transfectée ou infectée comprenant le peptide à composant de sphéron, ledit animal est un animal non-humain.

2. Procédé selon la revendication 1, comprenant:
la transformation, la transfection ou l'infection des cellules comprenant le peptide à composant de sphéron;
l'administration du composé d'essai aux cellules transformées, transfectées ou infectées; et
l'identification des composés de test qui améliorent les effets du peptide à composant de sphéron.

3. Procédé selon la revendication 1 ou 2, dans lequel la concentration du peptide à composant de sphéron dans les cellules transformées, transfectées ou infectées est suffisante pour réduire la viabilité des cellules.

4. Procédé selon l'une quelconque des revendications 1 à 3, dans lequel les cellules transformées, transfectées ou infectées sont cultivées *in vitro.*

5. Procédé selon l'une quelconque des revendications **1 à 3,** dans lequel les cellules transformées, transfectées ou infectées sont cultivées *in vivo.*

6. Procédé selon l'une quelconque des revendications 1 à 5, dans lequel le composé d'essai est administré à un animal non-humain ayant été transfecté par les cellules transformées, transfectées ou Infectées.

7. Procédé selon la revendication 6, comprenant en outre:
la préparation d'un modèle animal non-humain par injection dans le cerveau d'un animal non-humain d'un gène exprimant un ou plusieurs des peptides à composant de sphéron; et
l'induction du gène à exprimer le peptide à composant de sphéron.

8. Procédé selon la revendication 7, dans lequel le gène est injecté dans le cerveau d'un animal non-humain conjointement avec un vecteur.

9. Procédé selon la revendication 7 ou 8, comprenant en outre:
la préparation de modèles animaux non-humains, chaque modèle animal non-humain ayant un gène dans son cerveau, lequel gène exprime un ou plusieurs des peptides à composant de sphéron;
l'induction du gène, si présent, à exprimer le peptide à composant de sphéron;
l'administration d'un composé d'essai au groupe de modèles animaux non-humains;
le sacrifice des animaux non-humains;
la mesure de la proportion de peptides à composant de sphéron isolés présents dans le cerveau de l'animal non-humain sacrifié, et/ou la mesure du taux de cellules viables dans ou autour du locus du gène, et
la sélection des composés de test qui réduisent la proportion des peptides à composant de sphéron isolés, comparés aux contrôles, et/ou par sélection des composés de test ayant un taux de cellules viables plus élevé dans ou autour du locus du gène, comparé aux contrôles auxquels aucun composé d'essai n'a été administré.

10. Modèle animal non-humain comprenant un animal non-humain ayant un gène inséré dans son cerveau, le gène exprimant ainsi un ou plusieurs peptides à composant de sphéron autre que le composant de sphéron responsable directement pour la formation d'une plaque amyloïde,
dans lequel le peptide à composant de sphéron autre que le composant de sphéron responsable directement pour la formation d'une plaque amyloïde est choisi dans le groupe constitué de:
GEAAGAVQELAR (SEQ ID NO. 1);
GLSAASPPLAETGAPR (SEQ ID NO. 2);
ARAEAQEAEDQQAR (SEQ ID NO. 3);
ALAHLLEAERQER (SEQ ID NO. 5);
AADHDVGSELPPEGVLGALLR (SEQ ID NO. 6);
LETPAPQVPAR (SEQ ID NO. 7);
ILAGSADSEGVAAPR (SEQ ID NO. 8);
ARPVKEPRGLSAASPPLAETGAPRRF (SEQ ID NO. 9);
ARPVKEP (SEQ ID NO. 10);
GLSAASPPLAETGAPRRF (SEQ ID NO. 11);
AADHDVGSELPPEGVLGALLRVKRLETPAPQVPA (SEQ ID NO. 12);
AADHDVGSELPPEGVLGALLRV (SEQ ID NO. 13);
LETPAPQVPA (SEQ ID NO. 14);
RRSVPRGEAAG (SEQ ID NO. 15);
VLAQLLRVWGAPRNSD (SEQ ID NO. 16);
PALGLDDDPDAPAAQLAR (SEQ ID NO. 17);
LARALLRARLDPAALAA (SEQ ID NO. 18);
QLVPAPVPAAALRPRPPVYDD (SEQ ID NO. 19);
GPAGPDAEEAGDE (SEQ ID NO. 20);
TPDVDPELLRYLLGR (SEQ ID NO. 21); et
LLRVKR (SEQ ID NO. 22).

11. Modèle animal non-humain selon la revendication 10, produit par:
préparation d'un gène exprimant un ou plusieurs des peptides à composant de sphéron autre que le composant de sphéron responsable directement pour la formation d'une plaque amyloïde;
injection du gène dans le cerveau de l'animal non-humain, et
induction du gène exprimant un ou plusieurs des peptides à composant de sphéron autre que le composant de sphéron responsable directement pour la formation d'une plaque amyloïde.

12. Lignée cellulaire transformée, transfectée ou infectée comprenant des cellules recombinantes qui expriment une séquence d'acide nucléaire codant pour un peptide à composant de sphéron autre que le composant de sphéron responsable pour la formation d'une plaque amyloïde, dans laquelle le peptide à composant de sphéron autre que le composant de sphéron responsable pour la formation d'une plaque amyloïde est choisi dans le groupe constitué de:
GEAAGAVQELAR (SEQ ID NO. 1);
GLSAASPPLAETGAPR (SEQ ID NO. 2);
ARAEAQEAEDQQAR (SEQ ID NO. 3);
ALAHLLEAERQER (SEQ ID NO. 5);
AADHDVGSELPPEGVLGALLR (SEQ ID NO. 6);
LETPAPQVPAR (SEQ ID NO. 7);
ILAGSADSEGVAAPR (SEQ ID NO. 8);
ARPVKEPRGLSAASPPLAETGAPRRF (SEQ ID NO. 9);
ARPVKEP (SEQ ID NO. 10);
GLSAASPPLAETGAPRRF (SEQ ID NO. 11);
AADHDVGSELPPEGVLGALLRVKRLETPAPQVPA (SEQ ID NO. 12);
AADHDVGSELPPEGVLGALLRV (SEQ ID NO. 13);
LETPAPQVPA (SEQ ID NO. 14);
RRSVPRGEAAG (SEQ ID NO. 15);
VLAQLLRVWGAPRNSD (SEQ ID NO. 16);
PALGLDDDPDAPAAQLAR (SEQ ID NO. 17);
LARALLRARLDPAALAA (SEQ ID NO. 18);
QLVPAPVPAAALRPRPPVYDD (SEQ ID NO. 19);
GPAGPDAEEAGDE (SEQ ID NO. 20);
TPDVDPELLRYLLGR (SEQ ID NO. 21); et
LLRVKR (SEQ ID NO. 22).

13. Lignée cellulaire selon la revendication 12, dans laquelle la séquence d'acide nucléaire comprend au moins un polynucléotide codant pour l'une quelconque des SEQ ID NOS: 1-3 et 5-22.

14. Lignée cellulaire selon la revendication 13, dans laquelle la séquence d'acide nucléaire comprend au moins un polynucléotide codant pour l'une quelconque des SEQ ID NOS: 1-3 et 5-9.

15. Modèle animal non-humain selon l'une quelconque des revendications 12 à 14, dans lequel le peptide à composant de sphéron autre que le composant de sphéron responsable directement pour la formation d'une plaque amyloïde est choisi dans le groupe constitué des SEQ ID NOS: 1-3 et 5-9.

16. Composition comprenant au moins un anticorps, un fragment d'anticorps ou une chaîne courte d'anticorps se fixant à un peptide à composant de sphéron choisi dans le groupe constitué de:
GEAAGAVQELAR (SEQ ID NO. 1);
GLSAASPPLAETGAPR (SEQ ID NO. 2);
ARAEAQEAEDQQAR (SEQ ID NO. 3);
ALAHLLEAERQER (SEQ ID NO. 5);
AADHDVGSELPPEGVLGALLR (SEQ ID NO. 6);
LETPAPQVPAR (SEQ ID NO. 7);
ILAGSADSEGVAAPR (SEQ ID NO. 8);
ARPVKEPRGLSAASPPLAETGAPRRF (SEQ ID NO. 9);
ARPVKEP (SEQ ID NO. 10);
GLSAASPPLAETGAPRRF (SEQ ID NO. 11);
AADHDVGSELPPEGVLGALLRVKRLETPAPQVPA (SEQ ID NO. 12);
AADHDVGSELPPEGVLGALLRV (SEQ ID NO. 13);
LETPAPQVPA (SEQ ID NO. 14);
RRSVPRGEAAG (SEQ ID NO. 15);
VLAQLLRVWGAPRNSD (SEQ ID NO. 16);
PALGLDDDPDAPAAQLAR (SEQ ID NO. 17);
LARALLRARLDPAALAA (SEQ ID NO. 18);
QLVPAPVPAAALRPRPPVYDD (SEQ ID NO. 19);
GPAGPDAEEAGDE (SEQ ID NO. 20);
TPDVDPELLRYLLGR (SEQ ID NO. 21); et
LLRVKR (SEQ ID NO. 22);
et un tampon pharmaceutiquement acceptable, un diluent, un adjuvant, un porteur ou leur mélange.

17. Composition selon la revendication 16 pour l'utilisation comme médicament.


### SEQUENCE LISTING

<110> NYMOX CORPORATION
   AVERBACK, Paul
<120> SPHERON COMPONENTS USEFUL IN DETERMINING COMPOUNDS CAPABLE OF TREATING SYMPTOMS OF ALZHEIMER'S DISEASE, AND TREATMENTS AND ANIMAL MODELS PRODUCED THEREFROM
<130> 10107-65
<140> PCT/CA03/00295
   <141> 2003-03-04
<150> US 60/361,302
   <151> 2002-03-04
<160> 29
<170> PatentIn Ver. 2.1
<210> 1
   <211> 12
   <212> PRT
   <213> Homo sapiens
<400> 1
   Gly Glu Ala Ala Gly Ala Val Gln Glu Leu Ala Arg
    1               5                  10
<210> 2
   <211> 16
   <212> PRT
   <213> Homo sapiens
<400> 2
   Gly Leu Ser Ala Ala Ser Pro Pro Leu Ala Glu Thr Gly Ala Pro Arg
    1               5                  10                  15
<210> 3
   <211> 14
   <212> PRT
   <213> Homo sapiens
<400> 3
   Ala Arg Ala Glu Ala Gln Glu Ala Glu Asp Gln Gln Ala Arg
   1 5 10
<210> 4
   <211> 7
   <212> PRT
   <213> Homo sapiens
<400> 4
   Val Leu Ala Gln Leu Leu Arg
   1 5
<210> 5
   <211> 13
   <212> PRT
   <213> Homo sapiens
<400> 5
   Ala Leu Ala His Leu Leu Glu Ala Glu Arg Gln Glu Arg
   1 5 10
<210> 6
   <211> 21
   <212> PRT
   <213> Homo sapiens
<400> 6
   Ala Ala Asp His Asp Val Gly Ser Glu Leu Pro Pro Glu Gly Val Leu
   1 5 10 15
   Gly Ala Leu Leu Arg
   20
<210> 7
   <211> 11
   <212> PRT
   <213> Homo sapiens
<400> 7
   Leu Glu Thr Pro Ala Pro Gln Val Pro Ala Arg
   1 5 10
<210> 8
   <211> 15
   <212> PRT
   <213> Homo sapiens
<400> 8
   Ile Leu Ala Gly Ser Ala Asp Ser Glu Gly Val Ala Ala Pro Arg
   1               5                   10                  15
<210> 9
   <211> 26
   <212> PRT
   <213> Homo sapiens
<400> 9
   Ala Arg Pro Val Lys Glu Pro Arg Gly Leu Ser Ala Ala Ser Pro Pro
   1               5                   10                  15
   Leu Ala Glu Thr Gly Ala Pro Arg Arg Phe
               20                  25
<210> 10
   <211> 7
   <212> PRT
   <213> Homo sapiens
<400> 10
   Ala Arg Pro Val Lys Glu Pro
   1               5
<210> 11
   <211> 18
   <212> PRT
   <213> Homo sapiens
<400> 11
   Gly Leu Ser Ala Ala Ser Pro Pro Leu Ala Glu Thr Gly Ala Pro Arg
   1               5                   10                  15
   Arg Phe
<210> 12
   <211> 34
   <212> PRT
   <213> Homo sapiens
<400> 12
   Ala Ala Asp His Asp Val Gly Ser Glu Leu Pro Pro Glu Gly Val Leu
   1 5 10 15
   Gly Ala Leu Leu Arg Val Lys Arg Leu Glu Thr Pro Ala Pro Gln Val
   20 25 30
   Pro Ala
<210> 13
   <211> 22
   <212> PRT
   <213> Homo sapiens
<400> 13
   Ala Ala Asp His Asp Val Gly Ser Glu Leu Pro Pro Glu Gly Val Leu
   1 5 10 15
   Gly Ala Leu Leu Arg Val
   20
<210> 14
   <211> 10
   <212> PRT
   <213> Homo sapiens
<400> 14
   Leu Glu Thr Pro Ala Pro Gln Val Pro Ala
   1 5 10
<210> 15
   <211> 11
   <212> PRT
   <213> Homo sapiens
<400> 15
   Arg Arg Ser Val Pro Arg Gly Glu Ala Ala Gly
   1 5 10
<210> 16
   <211> 16
   <212> PRT
   <213> Homo sapiens
<400> 16
   Val Leu Ala Gln Leu Leu Arg Val Trp Gly Ala Pro Arg Asn Ser Asp
    1               5                  10                  15
<210> 17
   <211> 18
   <212> PRT
   <213> Homo sapiens
<400> 17
   Pro Ala Leu Gly Leu Asp Asp Asp Pro Asp Ala Pro Ala Ala Gln Leu
    1               5                  10                  15
   Ala Arg
<210> 18
   <211> 17
   <212> PRT
   <213> Homo sapiens
<400> 18
   Leu Ala Arg Ala Leu Leu Arg Ala Arg Leu Asp Pro Ala Ala Leu Ala
    1               5                  10                  15
   Ala
<210> 19
   <211> 21
   <212> PRT
   <213> Homo sapiens
<400> 19
   Gln Leu Val Pro Ala Pro Val Pro Ala Ala Ala Leu Arg Pro Arg Pro
    1               5                  10                  15
   Pro Val Tyr Asp Asp
               20
<210> 20
   <211> 13
   <212> PRT
   <213> Homo sapiens
<400> 20
   Gly Pro Ala Gly Pro Asp Ala Glu Glu Ala Gly Asp Glu
    1               5                  10
<210> 21
   <211> 15
   <212> PRT
   <213> Homo sapiens
<400> 21
   Thr Pro Asp Val Asp Pro Glu Leu Leu Arg Tyr Leu Leu Gly Arg
   1 5 10 15
<210> 22
   <211> 6
   <212> PRT
   <213> Homo sapiens
<400> 22
   Leu Leu Arg Val Lys Arg
   1 5
<210> 23
   <211> 12
   <212> PRT
   <213> Homo sapiens
<400> 23
   Val Leu Gly Ala Leu Leu Arg Val Lys Arg Leu Glu
   1 5 10
<210> 24
   <211> 5
   <212> PRT
   <213> Homo sapiens
<400> 24
   Glu Asn Lys His Gly
   1 5
<210> 25
   <211> 10
   <212> PRT
   <213> Homo sapiens
<400> 25
   Leu Asp Gly Ile Val Thr Asp Ala Ile Glu
   1 5 10
<210> 26
   <211> 7
   <212> PRT
   <213> Homo sapiens
<400> 26
   Ser Trp Gly Pro Asn Asp Asp
   1 5
<210> 27
   <211> 6
   <212> PRT
   <213> Homo sapiens
<400> 27
   Trp Ala Ser Gly Asn Gly
   1 5
<210> 28
   <211> 11
   <212> PRT
   <213> Homo sapiens
<400> 28
   Cys Asp Gly Tyr Thr Asp Ser Ile Tyr Thr Ile
   1 5 10
<210> 29
   <211> 5
   <212> PRT
   <213> Homo sapiens
<400> 29
   His Thr Gly Thr Ser
   1 5